(19) Europäisches Patentamt
European Patent Office
Office européen des brevets

(11) Veröffentlichungsnummer : **0 167 888**
**B1**

(12) # EUROPÄISCHE PATENTSCHRIFT

(45) Veröffentlichungstag der Patentschrift : **17.08.88**

(21) Anmeldenummer : **85107518.4**

(22) Anmeldetag : **18.06.85**

(51) Int. Cl.[4] : **C 07 F 9/65**, C 07 D239/36, C 07 D239/46, C 07 D239/52, C 07 D239/54

(54) **Verfahren zur Herstellung von Phosphorsäurederivaten und Zwischenprodukten.**

(30) Priorität : **27.06.84 DE 3423623**

(43) Veröffentlichungstag der Anmeldung : **15.01.86 Patentblatt 86/03**

(45) Bekanntmachung des Hinweises auf die Patenterteilung : **17.08.88 Patentblatt 88/33**

(84) Benannte Vertragsstaaten : **BE CH DE FR GB IT LI NL**

(56) Entgegenhaltungen :
**FR-A- 2 365 577**
**JOURNAL OF THE CHEMICAL SOCIETY, Dezember 1965, Seiten 7116-7119; D.T. HURST et al.: "Pyrimidines. Part XIV. Synthesis of 2,5-Dihydroxypyrimidine"**
**CHEMICAL ABSTRACTS, Band 86, Nr. 21, 23. Mai 1977, Seite 440, Nr. 155305c, Columbus, Ohio, US; H. KAEMMERER et al.: "Halogen as a readily cleavable protective group for reactive positions in phenols and phenolic compounds".**

(73) Patentinhaber : **BAYER AG**
**Konzernverwaltung RP Patentabteilung**
**D-5090 Leverkusen 1 Bayerwerk (DE)**

(72) Erfinder : **Maurer, Fritz, Dr.**
**Roeberstrasse 8**
**D-5600 Wuppertal 1 (DE)**

**EP 0 167 888 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung von insektiziden Pyrimidinylphosphorsäurederivaten, Zwischenprodukte welche für die Durchführung des Verfahrens verwendet werden können, sowie Verfahren zur Herstellung solcher Zwischenprodukte.

Es ist bereits bekannt, daß man bestimmte pestizide Phosphorsäurepyrimidinester erhält, wenn man entsprechende Phosphorsäureesterchloride mit 5-Hydroxypyrimidinen umsetzt (vgl. DE-OS 2 643 262 und DE-OS 2 706 127). Diese Herstellungsmethode ist jedoch wegen des Fehlens geeigneter Ausgangsverbindungen bzw. wegen unbefriedigender Herstellungsmethoden hierfür nur begrenzt anwendbar. Es besteht daher Bedarf an neuen Zwischenprodukten und ein entsprechendes Herstellungsverfahren für Phosphorsäurepyrimidinester.

Es wurde nun gefunden, daß man die Verbindungen der allgemeinen Formel I

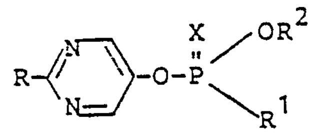

(I)

in welcher

R für Wasserstoff, für Alkoxy mit 1 bis 12 Kohlenstoffatomen, für Mono- oder Di-Alkylamino mit jeweils 1 bis 6 Kohlenstoffatomen im Alkylteil, für gegebenenfalls durch $C_1$-$C_4$-Alkoxy oder $C_1$-$C_4$-Alkylsulfonyl substituiertes Alkyl mit 1 bis 12 Kohlenstoffatomen, für gegebenenfalls durch $C_1$-$C_4$-Alkyl substituiertes Cycloalkyl mit 3 bis 8 Kohlenstoffatomen, und für gegebenenfalls durch $C_1$-$C_4$-Alkyl, $C_1$-$C_4$-Alkoxy oder $C_1$-$C_4$-Alkylsulfonyl substituiertes Aryl mit 6 bis 10 Kohlenstoffatomen steht,

$R^1$ für Reste aus der Reihe Alkyl, Alkoxy, Alkylthio mit jeweils 1 bis 6 Kohlenstoffatomen, für Mono- oder Dialkylamino mit 1 bis 6 Kohlenstoffatomen je Alkylgruppe und für Phenyl steht,

$R^2$ für Alkyl mit 1 bis 6 Kohlenstoffatomen steht,

wobei die Reste $R^1$ und $R^2$ durch 1 bis 3 gleiche oder verschiedene Substituenten aus der Reihe Alkyl (gilt nicht für die Fälle, in welchen $R^1$ bzw. $R^2$ für Alkyl steht), Alkoxy oder Alkylthio mit jeweils 1 bis 4 Kohlenstoffatomen oder Halogen, Cyano und Nitro substituiert sein können, und

X für Sauerstoff oder Schwefel steht, durch die Umsetzung der Verbindungen der allgemeinen Formel IV

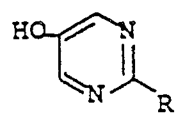

(IV)

in welcher R die oben angegebene Bedeutung hat, mit Verbindungen der allgemeinen Formel V

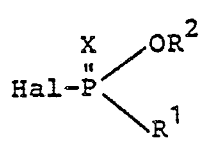

(V)

in welcher

Hal für Halogen steht und

X, $R^1$ und $R^2$ die oben angegebene Bedeutung haben,

gegebenenfalls in Gegenwart eines Säurebindemittels und gegebenenfalls in Gegenwart eines Lösungsmittels, mit nachfolgender Isolierung der Verbindungen der allgemeinen Formel I (Schritt (c)), dadurch gekennzeichnet, daß man die Verbindungen der allgemeinen Formel IV erhält, indem man

(a) Verbindungen der allgemeinen Formel II

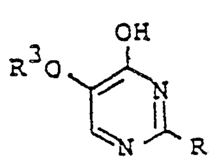

(II)

in welcher

R die oben angegebene Bedeutung hat,

$R^3$ für Wasserstoff oder eine Gruppierung

steht, wobei

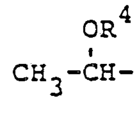

$R^4$ für Alkyl mit 1 bis 4 Kohlenstoffatomen steht, mit Halogenierungsmitteln in Gegenwart von N,N-disubstituierten Amiden und gegebenenfalls in Gegenwart von Verdünnungsmitteln bei Temperaturen zwischen 10 °C und 80 °C zu den Verbindungen der allgemeinen Formel III

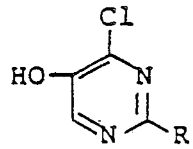

(III)

in welcher R die oben angegebene Bedeutung hat, umsetzt und anschließend

(b) die Verbindungen der allgemeinen Formel III, gegebenenfalls nach ihrer Isolierung, mit Wasserstoff in Gegenwart von Hydrierungskatalysatoren, in Gegenwart von Säureakzeptoren und in Gegenwart von Verdünnungsmitteln, bei Temperaturen zwischen 20 °C und 150 °C zu den Verbindungen der allgemeinen Formel IV umsetzt und diese zur weiteren Umsetzung mit den Verbindungen der allgemeinen Formel V gegebenenfalls isoliert.

In einer Variante des obigen Verfahrens ist es möglich, die Herstellung der Verbindungen der allgemeinen Formel II (bzw. VII) vorzuschalten, wobei dann diese gegebenenfalls ohne ihre Isolierung eingesetzt werden können.

Gemäß diesem Verfahren ist es möglich, die Verbindungen der Formel I auf einfache Weise in guter Reinheit und Ausbeute herzustellen. Das Verfahren ist im Hinblick auf die Art der gewünschten substituenten sehr breit anwendbar. Weiterhin sind die als Zwischenprodukte einzusetzenden Verbindungen stabil und können gut gelagert und gehandhabt werden.

Bevorzugte Substituenten bzw. Bereiche der in den oben und nachfolgend erwähnten Formeln aufgeführten Reste werden im folgenden erläutert :

Alkoxy R steht für geradkettiges oder verzweigtes Alkoxy mit 1 bis 12, insbesondere 1 bis 6 und besonders bevorzugt 1 bis 4 Kohlenstoffatomen. Beispielhaft seien Methoxy, Ethoxy, n-Propoxy, i-Propoxy, n-Butoxy, i-Butoxy, sec.-Butoxy und tert.-Butoxy genannt.

Mono- oder Di-Alkylamino R steht für eine Aminogruppe mit 1 oder 2 Alkylgruppen, vorzugsweise 2 Alkylgruppen, welche jeweils geradkettig oder verzweigt sein können und 1 bis 6, insbesondere 1 bis 3 Kohlenstoffatome enthalten, wobei Methyl, Ethyl, n- und i-Propyl genannt seien. Beispielhaft seien Dimethylamino, Diethylamino, Di-n-propylamino und Di-i-propylamino aufgeführt.

Als Alkyl R steht geradkettiges oder verzweigtes Alkyl mit 1 bis 12, insbesondere 1 bis 6 und besonders bevorzugt 1 bis 4 Kohlenstoffatomen. Beispielhaft seien Methyl, Ethyl, n-Propyl, i-Propyl, n-Butyl, sec.-Butyl, i-Butyl, tert.-Butyl, n-Pentyl, i-Pentyl und tert.-Pentyl genannt.

Das Cycloalkyl R enthält 3 bis 8, insbesondere 3, 5 oder 6 Kohlenstoffatomen. Beispielhaft seien gegebenenfalls substituiertes Cyclopropyl, Cyclobutyl, Cyclopentyl, Cyclohexyl und Cycloheptyl genannt.

Als Aryl R steht Aryl mit 6 bis 10 Kohlenstoffatomen im Arylteil. Beispielhaft seien gegebenenfalls substituiertes Phenyl oder Naphthyl, insbesondere Phenyl genannt.

Die in der Definition von R genannten substituierten Reste können einen oder mehrere, vorzugsweise 1 bis 3, insbesondere 1 oder 2 gleiche oder verschiedene Substituenten tragen. Als Substituenten für Alkyl und Aryl seien aufgeführt : Alkoxy und Alkylsulfonyl mit 1 bis 4 Kohlenstoffatomen, wie Methoxy, Ethoxy, n-Propoxy, i-Propoxy, n-Butoxy, i-Butoxy, sec.-Butoxy, tert.-Butoxy, Methylsulfonyl, Ethylsulfonyl, n-Propylsulfonyl, i-Propylsulfonyl, n-Butylsulfonyl, i-Butylsulfonyl und tert.-Butylsulfonyl.

Als Arylsubstituenten und Cycloalkylsubstituenten kommt außerdem noch $C_1$-$C_4$-Alkyl in Frage, wie Methyl, Ethyl, n-Propyl, i-Propyl, n-Butyl, i-Butyl, sec.-Butyl und tert.-Butyl.

Vorzugsweise steht

R für Wasserstoff, Alkoxy mit 1 bis 6 Kohlenstoffatomen, Mono- oder Dialkylamino mit jeweils 1 bis 4 Kohlenstoffatomen im Alkylteil oder für gegebenenfalls durch Methoxy, Ethoxy, Methylsulfonyl oder Ethylsulfonyl substituiertes Alkyl mit 1 bis 6 Kohlenstoffatomen, für gegebenenfalls durch Methyl oder Ethyl substituiertes Cycloalkyl mit 3 bis 6 Kohlenstoffatomen, und für gegebenenfalls durch Methyl, Ethyl, Methoxy, Ethoxy, Methylsulfonyl oder Ethylsulfonyl substituiertes Phenyl.

Ganz besonders bevorzugt steht

R für Methyl, Isopropyl und t-Butyl.

Die Alkylgruppen $R^1$ und $R^2$ enthalten 1 bis 6, insbesondere 1 bis 4 und besonders bevorzugt 1 oder 2 Kohlenstoffatome. Beispielhaft seien Methyl, Ethyl, n- und i-Propyl, n-, i-, s- und t-Butyl genannt.

Die Alkylgruppen der Alkyl- und Dialkylaminogruppen $R^1$ haben die für die Alkylgruppen $R^1$ und $R^2$

oben angegebene Bedeutung. Beispielhaft seien Methyl-, Ethyl-, n- und i-Propylamino sowie Dimethyl-, Diethyl- und Methyl-ethylamino aufgeführt.

Die Alkoxy- und Alkylthioreste $R^1$ enthalten 1 bis 6, insbesondere 1 bis 4 und besonders bevorzugt 1 oder 2 Kohlenstoffatome. Beispielhaft seien Methoxy, Ethoxy, n- und i-Propoxy sowie Methylthio, Ethylthio und n- und i-Propylthio genannt.

Die gegebenenfalls substituierten Reste $R^1$ und $R^2$ können 1 bis 3, insbesondere 1 oder 2 gleiche oder verschiedene der folgenden Substituenten tragen : Alkyl (gilt nicht für die Falle in welchen $R^1$ bzw. $R^2$ für Alkyl steht) mit 1 bis 4, insbesondere 1 oder 2 Kohlenstoffatomen, wie Methyl, Ethyl, n- und i-Propyl, und n-, i-, s- und t-Butyl ; Alkoxy mit 1 bis 4, insbesondere 1 oder 2 Kohlenstoffatomen, wie Methoxy, Ethoxy, n- und i-Propyloxy und n-, i-, s- und t-Butyloxy ; Alkylthio mit 1 bis 4, insbesondere 1 oder 2 Kohlenstoffatomen, wie Methylthio, Ethylthio, n- und i-Propylthio und n-, i-, s- und t-Butylthio ; Halogen, vorzugsweise Fluor, Chlor, Brom und Jod, insbesondere Chlor und Brom ; Cyano und Nitro.

Alkyl $R^4$ steht für $C_1$-$C_4$-, insbesondere $C_1$-$C_2$-Alkyl, wobei beispielhaft Methyl, Ethyl, n- und i-Propyl sowie n-, i-, s- und t-Butyl genannt seien.

Hal in der allgemeinen Formel V steht für Fluor, Chlor, Brom und Jod, vorzugsweise für Fluor, Chlor und Brom, insbesondere für Chlor.

Die im Verfahrensschritt (a) einzusetzenden Verbindungen der Formel II, in welcher $R^3$ für Wasserstoff steht, sind bekannt und/oder lassen sich in einfacher Weise nach bekannten Methoden herstellen, aus 5-Alkoxy-4-hydroxypyrimidinen der Formel VI

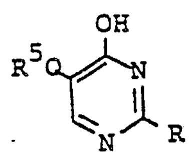

(VI)

in welcher

R die oben angegebenen Bedeutungen hat und

$R^5$ für $C_1$-$C_4$-Alkyl steht,

und starken Säuren wie z. B. Bromwasserstoffsäure oder konzentrierter Salzsäure bei Temperaturen zwischen 20 °C und 140 °C (vgl. J. Chem. Soc. 1963, 5590 und die Herstellungsbeispiele).

Die Verbindungen der Formel VI sind bekannt und/oder lassen sich nach bekannten Methoden herstellen (vgl. DE-OS 2 639 256 und die Herstellungsbeispiele). Sie können nach der gleichen Methode auch aus den unten beschriebenen Verbindungen der allgemeinen Formel VII erhalten werden.

Die beim Verfahrensschritt (a) einzusetzenden Verbindungen der Formel II, in welchen $R^3$ für die Gruppe

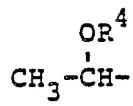

steht, sind neu und werden im folgenden mit der allgemeinen Formel VII beschrieben

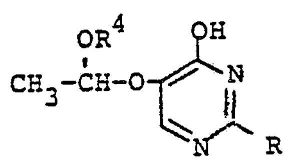

(VII)

in welcher R und $R^4$ die oben angegebene Bedeutung haben.

Die Verbindungen der allgemeinen Formel VII sowie das folgende Verfahren zu ihrer Herstellung sind Teil der vorliegenden Erfindung.

Es wurde gefungen, daß man die die neuen 4-Hydroxy-pyrimidin-Derivate der allgemeinen Formel VII

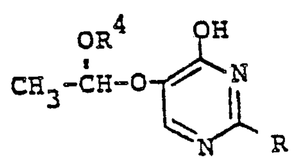

(VII)

in welcher R und $R^4$ die oben angegebene Bedeutung haben, erhält, wenn man Hydroxyessigsäureester der allgemeinen Formel VIII

$$HO—CH_2COOR^6 \qquad (VIII)$$

in welcher $R^6$ für $C_1$-$C_4$-Alkyl steht, mit Vinylethern der Formel IX

$$CH_2 = CH—OR^4 \qquad (IX)$$

in welcher $R^4$ die oben angegebene Bedeutung hat,
in Gegenwart von Katalysatoren umsetzt, weiterhin mit Ameisensäureestern der Formel X

$$HCOOR^7 \qquad (X)$$

in welcher $R^7$ für $C_1$-$C_4$-Alkyl steht,
in Gegenwart einer Base, reagieren läßt und anschließend mit Amidin-Hydrochloriden der allgemeinen Formel XI

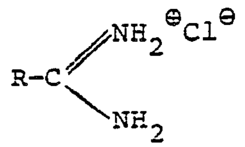

(XI)

in welcher R die oben angegebene Bedeutung hat,
in Gegenwart von Basen und in Gegenwart von Verdünnungsmitteln bei Temperaturen zwischen 15 °C und 60 °C umsetzt.

Überraschenderweise kann man nach dem erfindungsgemäßen Verfahren die neuen 4-Hydroxy-pyrimidin-Derivate der allgemeinen Formel VII in guten Ausbeuten und in hoher Reinheit erhalten, da zu erwarten war, daß eine Aneinanderreihung der obigen Reaktionsschritte ohne Isolierung und Reinigung der Zwischenprodukte nicht zu den gewünschten Produkten oder wegen Nebenreaktionen in einzelnen Stufen nur zu geringen Ausbeuten an verunreinigten Verbindungen führen würde.

Die neuen Verbindungen der allgemeinen Formel VII sind somit nach dem erfindungsgemäßen Verfahren sehr leicht zugänglich und für den Einsatz im Verfahrensschritt (a) in besonderem Maße geeignet.

Verwendet man beim erfindungsgemäßen Verfahren zur Herstellung der Verbindungen der allgemeinen Formel VII Glykolsäurebutylester, Ethylvinylether, Ameisensäure und Isobutyramidin-Hydrochlorid als Ausgangsstoffe, so kann die Reaktion durch das folgende Formelschema skizziert werden :

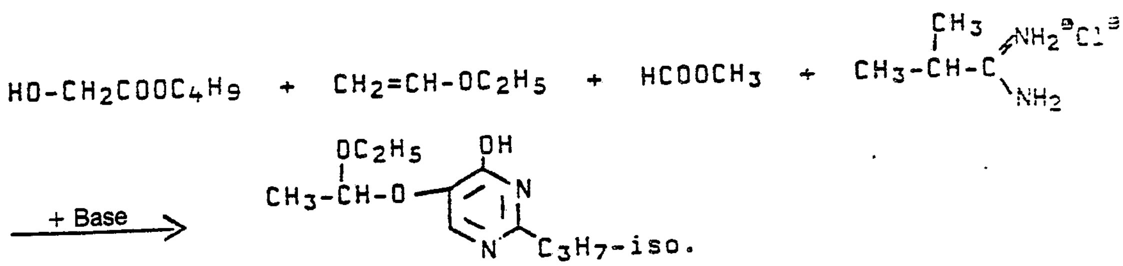


Die beim erfindungsgemäßen Verfahren als Ausgangsstoffe zu verwendenden Hydroxyessigsäureester sind durch die Formel VIII definiert.

Als Beispiele für die Verbindungen der Formel VIII seien die folgenden Verbindungen genannt : Methyl-, Ethyl-, n-Propyl-, i-Propyl-, n-Butyl-, i-Butyl-, sec.-Butyl und tert.-Butyl-ester der Hydroxyessigsäure.

Die außerdem als Ausgangsstoffe für das erfindungsgemäße Verfahren zu verwendenden Vinylether sind durch die Formel IX definiert.

Als Beispiele für die Verbindungen der Formel IX seien genannt : Methyl-, Ethyl-, n-Propyl-, i-Propyl-, n-Butyl-, i-Butyl-, sec.-Butyl- und tert.-Butyl-vinylether.

Die weiterhin als Ausgangsstoffe zu verwendenden Ameisensäureester sind durch die Formel X definiert.

Als Beispiele für die Verbindungen der Formel X seien genannt : Methyl-, Ethyl-, n-Propyl-, i-Propyl-, n-Butyl-, i-Butyl-, sec.-Butyl- und tert.-Butyl-ameisensäureester.

Die weiterhin als Ausgangsstoffe für das erfindungsgemäße Verfahren zu verwendenden Amidin-Hydrochloride sind durch die Formel XI definiert.

Als Beispiele für die Verbindungen der Formel XI seien die folgenden Verbindungen genannt :

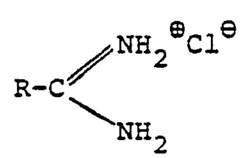

(XI)

Tabelle 1

| R | R |
|---|---|
| H | $OC_2H_5$ |
| $CH_3$ | $OC_3H_7$-n |
| $C_2H_5$ | $OC_3H_7$-iso |
| $C_3H_7$-n | $-CH_2OCH_3$ |
| $C_3H_7$-iso | $-CH_2CH_2OCH_3$ |
| $C_4H_9$-n | $-CH_2OC_2H_5$ |
| $C_4H_9$-iso | $-CH_2CH_2OC_2H_5$ |
| $C_4H_9$-sec | $-CH_2SO_2CH_3$ |
| $C_4H_9$-tert | $-CH_2CH_2SO_2CH_3$ |
| $C_5H_{11}$-n | $-CH_2CH_2SO_2C_2H_5$ |
| $C_5H_{11}$-tert | $-N(CH_3)_2$ |
| $OCH_3$ | $-N(C_2H_5)_2$ |
| 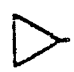 | 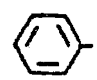 |
| 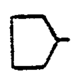 | 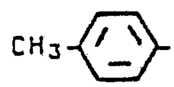 |
| 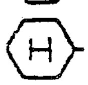 | |

Die Verbindungen der Formeln VIII, IX, X und XI sind bekannt und/oder lassen sich nach einfachen und bekannten Methoden herstellen (vgl. z. B. US-PS 4 012 506 ; DRP 584 840 ; Liebigs Ann. Chem. 601, 84 (1956) ; « Organic Functional Group Preparations » Vol. III, S. 205-240, Academic Press 1972).

Das erfindungsgemäße Verfahren zur Herstellung der Verbindungen der allgemeinen Formel VII wird bevorzugt in Gegenwart von Verdünnungsmitteln durchgeführt. Als Verdünnungsmittel kommen vorzugsweise in Frage :

Alkohole, wie Methanol, Ethanol, n- und i-Propanol, tert.-Butanol, aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glykoldimethylether und Diglykoldimethylether, Tetrahydrofuran und Dioxan, Ketone, wie Aceton, Methyl-ethyl-, Methylisopropyl- und Methylisobutylketon, Ester, wie Essigsäuremethylester und -ethylester, Nitrile, wie z. B. Acetonitril und Propionitril, Amide, wie z. B. Dimethylacetamid und N-Methyl-pyrrolidon, sowie Tetramethylensulfon.

Als Basen für das erfindungsgemäße Verfahren können praktisch alle üblicherweise verwendbaren Säurebindemittel eingesetzt werden. Hierzu gehören insbesondere : Alkali- und Erdalkalihydroxide, bzw. -oxide wie Natrium- und Kaliumhydroxid und insbesondere Lithiumhydroxid sowie Calciumoxid oder Calcium-hydroxid, Alkali- und Erdalkalicarbonate, wie Natrium-, Kalium- und Calciumcarbonat, Alkalialkoholate, wie Natrium-methylat, -ethylat und tert.-butylat, ferner aliphatische, aromatische oder heterocyclische Amine, wie Tri-ethylamin, Dimethylanilin, Dimethylbenzylamin, Pyridin, Diazabicyclooctan und Diazabicycloundecen.

Als Katalysatoren werden vorwiegend saure Katalysatoren wie organische Sulfonsäuren, vorzugsweise aromatische Sulfonsäuren, wie Toluolsulfonsäure eingesetzt.

Die Reaktionstemperatur kann innerhalb eines größeren Bereichs variiert werden. Im allgemeinen arbeitet man zwischen — 15 °C und + 70 °C, vorzugsweise bei — 10 °C bis + 60 °C. Das erfindungsgemäße Verfahren wird im allgemeinen bei Normaldruck durchgeführt.

Zur Durchführung des erfindungsgemäßen Verfahrens werden die Ausgangsstoffe gewöhnlich in äquimolaren Mengen eingesetzt. Ein Überschuß der einen oder anderen Reaktionskomponente bringt keine wesentlichen Vorteile. Die Aufarbeitung sowie die gegebenenfalls gewünschte Isolierung geschieht nach üblichen Methoden.

Im Verfahrensschritt (a) werden die Verbindungen der allgemeinen Formel II (bzw. VII) gegebenenfalls ohne ihre Isolierung in die neuen Verbindungen der allgemeinen Formel III umgewandelt. Die

Verbindungen der allgemeinen Formel III sowie das Verfahren zu ihrer Herstellung gemäß Verfahrensschritt (a) sind Teil der vorliegenden Erfindung.

Es wurde gefunden, daß man die neuen 4-Chlor-5-hydroxy-pyrimidine der allgemeinen Formel III

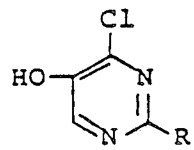

(III)

in welcher R die oben angegebene Bedeutung hat, erhält, wenn man Pyrimidin-Derivate der allgemeinen Formel II

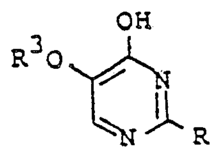

(II)

in welcher R und $R^3$ die oben angegebene Bedeutung haben, mit Halogenierungsmitteln in Gegenwart von N,N-disubstituierten Amiden und gegebenenfalls in Gegenwart von Verdünnungsmitteln bei Temperaturen zwischen 10 °C und 80 °C umsetzt.

Es ist als überraschend zu bezeichen, daß man nach dem erfindungsgemäßen Verfahren die neuen 4-Chlor-5-hydroxy-pyrimidine in guten Ausbeuten und in hoher Reinheit erhält, da zu erwarten war, daß unter den Reaktionsbedingungen auch der Rest —$OR^3$ angegriffen wird und/oder daß aufgrund der Gegenwart von substituierten Amiden z. B. Formylpyrimidine (« Vilsmeyer-Reaktion ») entstehen.

Verwendet man beim erfindungsgemäßen Verfahren 4,5-Dihydroxy-2-methyl-pyrimidin als Ausgangsstoff und Phosgen, in Gegenwart von Dimethylformamid als Halogenierungsmittel, so kann die Reaktion durch das folgende Formelschema skizziert werden :

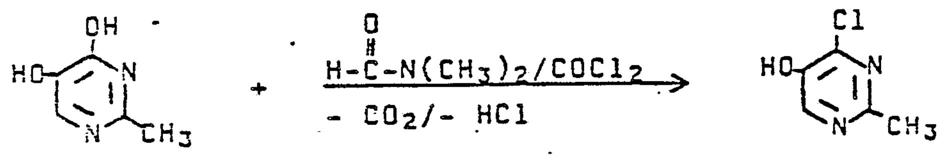


Verwendet man beim erfindungsgemäßen Verfahren 2-tert.-Butyl-5-(1-ethoxy-ethoxy)-4-hydroxy-pyrimidin als Ausgangsstoff und Phosgen in Gegenwart von Dimethylformamid als Halogenierungsmittel, so kann die Reaktion durch das folgende Formelschema skizziert werden :

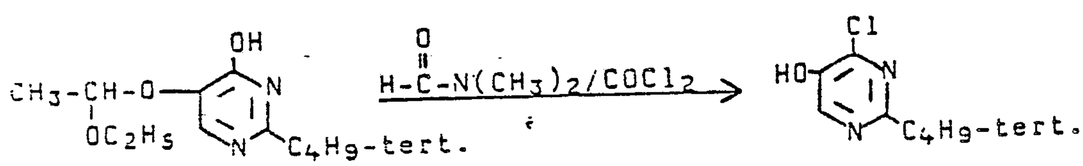


Als Beispiele für die Ausgangsverbindungen der allgemeinen Formel (II) seien die folgenden Verbindungen aufgeführt :

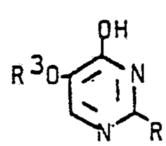

(II)

(Siehe Tabelle 2 Seite 8 f.)

Tabelle 2

$R^3 = H$, $CH_3-CH(OC_2H_5)-$ , $CH_3-CH(OCH_3)-$ oder $CH_3-CH(OC_3H_7)-$

| R | R |
|---|---|
| H | $OC_2H_5$ |
| $CH_3$ | $OC_3H_7$-n |
| $C_2H_5$ | $OC_3H_7$-iso |
| $C_3H_7$-n | $-CH_2OCH_3$ |
| $C_3H_7$-iso | $-CH_2CH_2OCH_3$ |
| $C_4H_9$-n | $-CH_2OC_2H_5$ |
| $C_4H_9$-iso | $-CH_2CH_2OC_2H_5$ |
| $C_4H_9$-sec | $-CH_2SO_2CH_3$ |
| $C_4H_9$-tert | $-CH_2CH_2SO_2CH_3$ |
| $C_5H_{11}$-n | $-CH_2CH_2SO_2C_2H_5$ |
| $C_5H_{11}$-tert | $-N(CH_3)_2$ |
| $OCH_3$ | $-N(C_2H_5)_2$ |
| 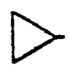 | 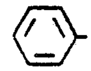 |
| 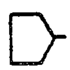 | 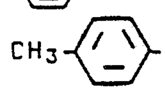 |
| 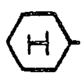 | |

Das erfindungsgemäße Verfahren zur Herstellung der Verbindungen der allgemeinen Formel III bzw. der Verfahrensschritt (a) wird bevorzugt in Gegenwart von Verdünnungsmitteln durchgeführt. Als Verdünnungsmittel kommen inerte organische Lösungsmittel in Frage. Hierzu gehören : Benzol, Chlorbenzol, O-Dichlorbenzol, Methylenchlorid, Ethylenchlorid, Chloroform, Tetrachlorkohlenstoff, Amide wie z. B. Dimethylformamid, N-Methylformamid und N-Methylpyrrolidon.

Als Halogenierungsmittel für das erfindungsgemäße Verfahren werden vorzugsweise verwendet : Phosphoroxychlorid, Phosphortrichlorid, Oxalylchlorid, Phosgen oder Thionylchlorid.

Als N,N-disubstituierte Amide kommen vorzugsweise in Frage : Dimethylformamid, N-Methylformanilid, N-Methylpyrrolidon oder N-Methylpiperidon.

Das erfindungsgemäße Verfahren wird im allgemeinen bei Temperaturen zwischen 10 °C und 80 °C durchgeführt. Bevorzugt wird der Bereich zwischen 20 °C und 60 °C. Die Umsetzungen werden im allgemeinen bei Normaldruck durchgeführt.

Zur Durchführung des erfindungsgemäßen Verfahrens wird auf 1 Mol der Verbindung der Formel II, 1 bis 3 Mol, vorzugsweise 1,2 bis 2 Mol Halogenierungsmittel und 1 bis 3 Mol, vorzugsweise 1,2 bis 2 Mol N,N-disubstituiertes Amid eingesetzt. Die Aufarbeitung und gegebenenfalls gewünschte Isolierung der Verbindungen der allgemeinen Formel III geschieht nach üblichen Methoden.

Als Beispiele für die erfindungsgemäß erhältlichen Verbindungen der Formel III seien die folgenden Verbindungen aufgeführt :

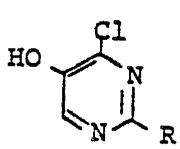

(III)

Tabelle 3

| R | R |
|---|---|
| H | $-CH_2CH_2OCH_3$ |
| $CH_3$ | $-CH_2OC_2H_5$ |
| $C_2H_5$ | $-CH_2CH_2OC_2H_5$ |
| $C_3H_7$-n | $-CH_2SO_2CH_3$ |
| $C_3H_7$-i | $-CH_2CH_2SO_2CH_3$ |
| $C_4H_9$-sec. | $-CH_2CH_2SO_2C_2H_5$ |
| $C_4H_9$-tert. | $-N(CH_3)_2$ |
| $C_5H_{11}$-n | $-N(C_2H_5)_2$ |
| $C_5H_{11}$-tert. | 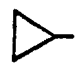 |
| $OCH_3$ | 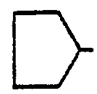 |
| $OC_2H_5$ | 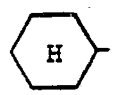 |

Tabelle 3 (Fortsetzung)

| R | R | R |
|---|---|---|
| $OC_3H_7$-n | 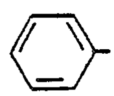 | $-CH_2OCH_3$ |
| $OC_3H_7$-i | 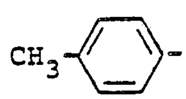 | |

Die in der Verfahrensstufe (c) einzusetzenden Verbindungen der allgemeinen Formel IV sind bekannt bzw. können nach allgemein bekannten Methoden hergestellt werden.

So ist es bereits bekannt, daß man 5-Hydroxy-pyrimidine erhält, wenn man 5-Methoxy-pyrimidine in Autoklaven, bei Temperaturen zwischen 180 °C und 200 °C unter basischen Bedingungen umsetzt (vgl. z. B. DE-OS 2 643 262 und Coll. Czech. Chem. Comm. 40, 1078 ff (1975)). Die Nachteile dieser Verfahren bestehen darin, daß die Ausbeuten und die Reinheit der Reaktionsprodukte häufig unbefriedigend sind und außerdem extreme Reaktionsbedingungen erforderlich sind.

Es ist außerdem bekannt, daß man die 5-Hydroxy-pyrimidine auch in Gegenwart von Alkalihydroxiden und Glykol aus 5-Methoxy-pyrimidinen herstellen kann. Bei diesem Verfahren sind Temperaturen von ca. 200 °C erforderlich. Weitere Nachteile sind die aufwendige Aufarbeitung der Endprodukte und die mäßigen Ausbeuten (vgl. z. B. J. Chem. Soc. 1960, 4590 ff und Chem. Ber. 95, 803 ff (1962)). Außerdem verlangt die Arbeitsweise in polaren hochsiedenden Lösungsmitteln wie Glykol besondere Anstrengungen zur Abwasserreinigung.

Es wurde gefunden, daß man 5-Hydroxy-pyrimidine der allgemeinen Formel IV

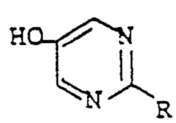

(IV)

in welcher R die oben angegebene Bedeutung hat, erhält, wenn man substituierte 4-Chlor-pyrimidin-Derivate der Formel III

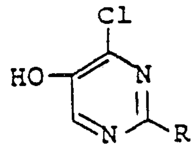

(III)

in welcher R die oben angegebenen Bedeutungen hat,
mit Wasserstoff in Gegenwart von Hydrierungskatalysatoren, in Gegenwart von Säureakzeptoren und in Gegenwart von Verdünnungsmitteln bei Temperaturen zwischen 20 °C und 150 °C umsetzt.

Überraschenderweise ist es möglich mit Hilfe dieses Verfahrens, welches dem Verfahrensschritt (b) entspricht und welches Teil der vorliegenden Erfindung ist, unter relativ milden Bedingungen die 5-Hydroxypyrimidine der allgemeinen Formel IV in guter Ausbeute und sehr hoher Reinheit zu erhalten. Weitere Vorteile des Verfahrens sind die Rückgewinnung der Katalysatoren und die Verwendung von preisgünstigen und umweltfreundlicheren Verdünnungsmitteln.

Verwendet man beispielsweise für das erfindungsgemäße Verfahren 4-Chlor-5-hydroxy-pyrimidin und Raney-Nickel als Katalysator, so kann die Reaktion durch das folgende Formelschema skizziert werden :

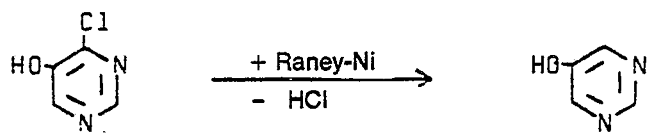


Für die Herstellung der Verbindungen der allgemeinen Formel IV aus den Verbindungen der allgemeinen Formel III wird vorzugsweise Wasser als Lösungsmittel verwendet.

Als Säureakzeptoren kommen für das erfindungsgemäße Verfahren alle üblicherweise verwendbaren anorganischen und organischen Basen in Betracht. Hierzu gehören vorzugsweise Alkalicarbonate, wie z. B. Natrium- und Kaliumcarbonat ; Alkalihydroxide, wie z. B. Natriumhydroxid ; Alkalialkoholate, wie z. B. Natrium und Kalium-methylat und -ethylat ; sowie niedere tertiäre Alkylamine, Cycloalkylamine und Aralkylamine, wie insbesondere Triethylamin.

Das erfindungsgemäße Verfahren wird in Gegenwart eines Hydrierungskatalysators durchgeführt. Es werden vorzugsweise neutrale Metallkatalysatoren wie Raney-Nickel, Raney-Cobalt oder Palladium gegebenenfalls auf üblichen Trägermaterialien, wie z. B. Aktivkohle, eingesetzt.

Die Reaktionstemperaturen können bei der Durchführung des erfindungsgemäßen Verfahrens in einem größeren Bereich variiert werden. Im allgemeinen arbeitet man zwischen 20 °C und 150 °C, vorzugsweise zwischen 20 °C und 100 °C, insbesondere zwischen 40 °C und 80 °C.

Das erfindungsgemäße Verfahren wird im allgemeinen bei erhöhtem Druck, vorzugsweise zwischen 5 und 60 bar, insbesondere zwischen 7 und 40 bar, durchgeführt.

Zur Durchführung des erfindungsgemäßen Verfahrens setzt man auf 1 Mol 4-Chlor-pyrimidin-Derivat der Formel III zwischen 1 und 5 Mol, vorzugsweise zwischen 1,2 und 3 Mol Säureakzeptor und zwischen 1 und 100 g, vorzugsweise zwischen 5 und 50 g Katalysator ein.

Die Ausgangsstoffe der Formel III, der Säureakzeptor, der Katalysator und das Verdünnungsmittel werden vermischt und während des Aufheizens auf die erforderliche Temperatur wird Wasserstoff eingedrückt. Bei konstanter Temperatur wird so lange Wasserstoff eingedrückt, bis die Druckkonstanz das Reaktionsende anzeigt.

Als Beispiele für die erfindungsgemäß erhältlichen Verbindungen der allgemeinen Formel IV seien aufgeführt :

Tabelle 4

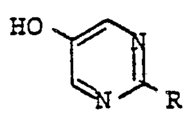

(IV)

| R | R |
|---|---|
| H | $OC_2H_5$ |
| $CH_3$ | $OC_3H_7$-n |
| $C_2H_5$ | $OC_3H_7$-iso |
| $C_3H_7$-n | $-CH_2OCH_3$ |
| $C_3H_7$-iso | $-CH_2CH_2OCH_3$ |

Tabelle 4 (Fortsetzung)

| R | R |
|---|---|
| $C_4H_9$-n | $-CH_2OC_2H_5$ |
| $C_4H_9$-iso | $-CH_2CH_2OC_2H_5$ |
| $C_4H_9$-sec | $-CH_2SO_2CH_3$ |
| $C_4H_9$-tert | $-CH_2CH_2SO_2CH_3$ |
| $C_5H_{11}$-n | $-CH_2CH_2SO_2C_2H_5$ |
| $C_5H_{11}$-tert | $-N(CH_3)_2$ |
| $OCH_3$ | $-N(C_2H_5)_2$ |
| 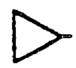 | 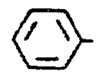 |
| 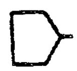 | 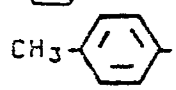 |
| 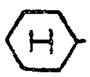 | |

Diese Verbindungen können beispielsweise in der Verfahrensstufe (c) eingesetzt werden.

In der Verfahrensstufe (c) werden die Verbindungen der allgemeinen Formel I aus den Verbindungen der allgemeinen Formeln IV und V erhalten.

Verwendet man in der Verfahrensstufe (c) beispielsweise O-Ethyl-O-isopropyl-thionophosphorsäurediesterchlorid und 5-Hydroxy-2-phenyl-pyrimidin als Ausgangsstoffe, so kann die entsprechende Reaktion durch das folgende Formelschema skizziert werden :

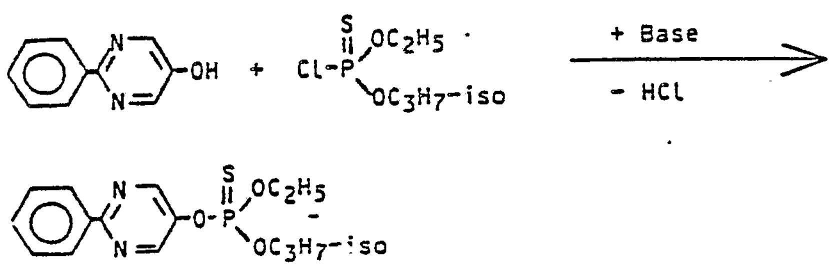


Die in der Verfahrensstufe (c) einzusetzenden Ausgangsstoffe der allgemeinen Formel V sind bekannt und nach literaturbekannten Verfahren und Methoden technisch gut herstellbar. Als Beispiele dafür seien im einzelnen genannt :

O,O-Dimethyl-, O,O-Diethyl-, O,O-Di-n-propyl, O,O-Di-iso-propyl, O,O-Di-n-butyl-, O,O-Di-iso-butyl-, O,O-Di-sec.-butyl, O-Methyl-O-ethyl-, O-Methyl-O-n-propyl-, O-Methyl-O-iso-propyl-, O-Methyl-O-n-butyl-, O-Methyl-O-iso-butyl-, O-Methyl-O-sec.-butyl-, O-Ethyl-O-n-propyl-, O-Ethyl-O-iso-propyl, O-Ethyl-O-n-butyl-, O-Ethyl-O-sec.-butyl-, O-Ethyl-O-iso-butyl-, O-n-Propyl-O-butyl- bzw. O-iso-Propyl-O-butylphosphorsäurediesterchlorid und die entsprechenden Thionoanalogen, ferner O,S-Dimethyl-, O,S-Diethyl-, O,S-Di-n-propyl-, O,S-Di-iso-propyl-, O,S-Di-n-butyl-, O,S-Di-iso-butyl-, O-Ethyl-S-n-propyl-, O-Ethyl-S-iso-propyl-, O-Ethyl-S-n-butyl-, O-Ethyl-S-sec.-butyl-, O-n-Propyl-S-ethyl-, O-n-Propyl-S-iso-propyl-, O-n-Butyl-S-n-propyl- und O-sec.-Butyl-S-ethylthiol-phosphorsäurediesterchlorid und die entsprechenden Thioanalogen, ferner O-Methyl-, O-Ethyl-, O-n-Propyl-, O-iso-Propyl-, O-n-Butyl-, O-iso-Butyl- bzw. O-sec.-Butyl-methan- bzw. -ethan-, -n-propan-, -iso-propan-, -n-butan-, -iso-butan-, -sec.-butan- bzw. -phenyl-phosphorsäureesterchlorid und die entsprechenden Thionoanalogen, und O-Methyl-N-methyl-, O-Methyl-N-ethyl-, O-Methyl-N-n-propyl-, O-Methyl-N-iso-propyl-, O-Ethyl-N-methyl-, O-Ethyl-N-ethyl-, O-Ethyl-N-n-propyl-, O-Ethyl-N-iso-propyl-, O-n-Propyl-N-methyl-, O-n-Propyl-N-ethyl-, O-n-Propyl-N-n-propyl-, O-n-Propyl-N-iso-propyl-, O-iso-Propyl-N-methyl-, O-iso-Propyl-N-ethyl-, O-iso-Propyl-N-n-propyl-, O-iso-Propyl-N-iso-propyl-, O-n-Butyl-N-methyl-, O-n-Butyl-N-ethyl-, O-n-Butyl-N-n-propyl-, O-n-Butyl-N-iso-propyl-, O-iso-Butyl-N-methyl-, O-iso-Butyl-N-ethyl-, O-iso-Butyl-N-n-propyl-, O-iso-Butyl-N-iso-propyl-, O-sec.-Butyl-N-methyl-, O-sec.-Butyl-N-ethyl-, O-sec.-Butyl-N-n-propyl- und O-sec.-Butyl-N-iso-propyl-phosphorsäuremonoesteramidchlorid und die entsprechenden Thionoanalogen.

Die Verfahrensstufe (c) zur Herstellung der Verbindungen der allgemeinen Formel I wird bevorzugt unter Mitverwendung geeigneter Lösungs- und Verdünnungsmittel durchgeführt. Als solche kommen praktisch alle inerten organischen Solventien in Frage. Hierzu gehören insbesondere aliphatische und

aromatische, gegebenenfalls chlorierte Kohlenwasserstoffe, wie Benzol, Toluol, Xylol, Benzin, Methylenchlorid, Chloroform, Tetrachlorkohlenstoff, Chlorbenzol oder Ether, wie Diethyl- und Dibutylether, Dioxan, ferner Ketone, beispielsweise Aceton, Methylethyl-, Methylisopropyl- und Methylisobutylketon, außerdem Nitrile, wie Aceto- und Propionitril.

Als Säureakzeptoren können alle üblichen Säurebindemittel Verwendung finden. Besonders bewährt haben sich Alkalicarbonate und -alkoholate, wie Natrium- und Kaliumcarbonat, Kalium-tert.-butylat, ferner aliphatische, aromatische oder heterocyclische Amine, beispielsweise Triethylamin, Trimethylamin, Dimethylanilin, Dimethylbenzylamin und Pyridin.

Die Reaktionstemperatur kann innerhalb eines größeren Bereichs variiert werden. Im allgemeinen arbeitet man zwischen 0 und 100 °C, vorzugsweise bei 20 bis 60 °C.

Die Umsetzung läßt man im allgemeinen bei Normaldruck ablaufen.

Zur Durchführung der Verfahrensstufe (c) setzt man die Ausgangsmaterialien meist in äquivalentem Verhältnis ein. Ein Überschuß der einen oder anderen Komponente bringt keine wesentlichen Vorteile. Die Reaktionspartner werden meist in einem der oben angeführten Lösungsmittel in Gegenwart eines Säurebindemittels vereinigt und bei erhöhter Temperatur zur Vervollständigung der Reaktion eine oder mehrere Stunden gerührt. Danach versetzt man die Mischung mit einem organischen Lösungsmittel, z. B. Toluol, und arbeitet die organische Phase in üblicher Weise durch Waschen, Trocknen und Abdestillieren des Lösungsmittels auf.

Die Verbindungen der allgemeinen Formel I fallen meist in Form von Ölen an, die sich häufig nicht unzersetzt destillieren lassen, jedoch durch sogenanntes « Andestillieren », d. h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Wie bereits mehrfach erwähnt, zeichnen sich die erfindungsgemäß erhältlichen Verbindungen der allgemeinen Formel I durch eine hervorragende insektizide, akarizide und nematizide Wirkung aus. Sie wirken gegen Pflanzen-, Hygiene- und Vorratsschädlinge und auf dem veterinärmedizinischen Sektor. Sie besitzen bei geringer Phytotoxizität sowohl eine gute Wirkung gegen saugende als auch fressende Insekten und Milben.

Aus diesem Grunde können die erfindungsgemäß erhältlichen Verbindungen der allgemeinen Formel I mit Erfolg im Pflanzenschutz sowie auf dem Hygiene-, Vorratsschutz- und Veterinärsektor als Schädlingsbekämpfungsmittel eingesetzt werden.

Viele der erfindungsgemäß erhältlichen Verbindungen und ihre Verwendung sind bekannt und werden beschrieben z. B. in DE-OS 2 643 262, US-PS 4 127 652, EP-A-0 009 566, US-PS 4 325 948, US-PS 4 444 764 und US-PS 4 429 125.

Wie bereits oben dargelegt, ist es nach dem erfindungsgemäßen Verfahren gemäß den Verfahrensstufen (a) bis (c) möglich, die wertvollen Verbindungen der allgemeinen Formel I in glatten Reaktionen auf einfache Weise herzustellen, wobei hervorragende Gesamtausbeuten erhalten werden. Das erfindungsgemäße Verfahren (a) bis (c) eröffnet in überraschender Weise durch die spezielle Kombination der Verfahrensschritte und durch den teilweisen Einsatz hierbei entstehender neuer Verbindungen einen Weg, welcher eine bisher nicht erreichbare kostengünstige Herstellung der Verbindungen der allgemeinen Formel I erlaubt. Da die einzelnen Zwischenprodukte stabil sind und vor allem im Falle ihrer Isolierung über längere Zeit bevorratet werden können, erlaubt das erfindungsgemäße Verfahren darüber hinaus eine außerordentliche Flexibilität in der Produktion, so daß bei rasch einsetzendem Bedarf der Endprodukte eine bedarfsgerechte Fertigung möglich ist, was insbesondere durch die klimatisch bedingten starken saisonalen Schwankungen auf dem Pflanzenschutzgebiet von sehr großer Bedeutung sein kann.

Im folgenden sollen die erfindungsgemäßen Verfahren (bzw. Verfahrensstufen) und Verbindungen durch die nachstehenden Herstellungsbeispiele erlaütert werden :

I Verfahren zur Herstellung der Verbindungen der allgemeinen Formel VII (bzw. II, mit $R^3 = CH_3\text{-}CHOR^4\text{-}$)

Beispiel I/1

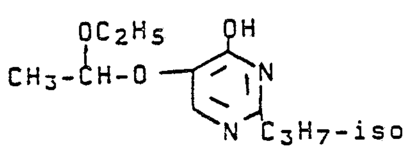

Eine Mischung aus 132 g (1 Mol) Glykolsäurebutylester, 72 g (1 Mol) Ethylvinylether und 0,3 g p-Toluolsulfonsäure läßt man unter schwachem Kühlen bei maximal 40 °C ausreagieren. Nach Ende der exothermen Reaktion wird 2 Stunden bei 40 °C nachgerührt, dann wird bei 20 °C erst 90 g (1,5 Mol) Ameisensäuremethylester und anschließend unter leichtem Kühlen bei 20 °C 62 g (1,15 Mol) Natriummethylat-Pulver portionsweise zugegeben. Das Reaktionsgemisch wird 1 1/2 Stunden bei 20 °C nachgerührt und anschließend mit 211 g (1 Mol) methanolischer Natriummethylat-Lösung und 122,5 g (1 Mol) Isobutyramidin-Hydrochlorid versetzt. Man rührt die Mischung ohne Kühlung 18 Stunden nach,

destilliert das Lösungsmittel im Vakuum ab und löst den Rückstand in 500 ml Wasser. Die Lösung wird im Vakuum bei 40 °C von restlichem organischen Lösungsmittel befreit und dann bei 5 °C mit 66 g (1,1 Mol) Eisessig versetzt. Das auskristallisierte Produkt wird abgesaugt und mit Wasser nachgewaschen.

Man erhält 181,7 g (80 % der Theorie) 5-(1-Ethoxy-ethoxy)-4-hydroxy-2-i-propyl-pyrimidin in Form eines farblosen Pulvers mit dem Schmelzpunkt 70 °C.

Analog Beispiel 1 werden z. B. die folgenden Verbindungen der Formel VII erhalten :

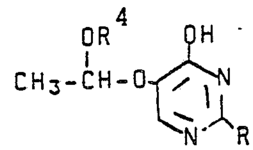

(VII)

Tabelle 5

| Beispiel-Nr. | $R^4$ | R | Ausbeute (% d.Th.) | Physikalische Konstante |
|---|---|---|---|---|
| I/2 | $C_2H_5$ | tert.-$C_4H_9$ | 83 | Fp: 104 - 105°C |
| I/3 | $C_2H_5$ | $-CH_2-S-CH_3$ | 98 | $n_D^{26}$: 1,5441 |
| I/4 | $C_2H_5$ | 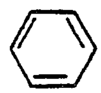 | 83 | Fp: 115°C |

Ia Verfahren zur Herstellung der Verbindungen der allgemeinen Formel II ($R^3$ = H)

Beispiel Ia/1

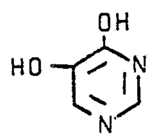

Eine Mischung aus 132 g (1 Mol) Glykolsäurebutylester, 72 g (1 Mol) Ethylvinylether und 0,3 g p-Toluolsulfonsäure läßt man unter schwachem Kühlen bei maximal 40 °C ausreagieren. Nach Ende der exothermen Reaktion wird 2 Stunden bei 40 °C nachgerührt, dann gibt man bei 20 °C erst 90 g (1,5 Mol) Ameisensäuremethylester und anschließend unter leichtem Kühlen bei 20 °C 62 g (1,15 Mol) Natrium-methylat-Pulver portionsweise zu. Das Reaktionsgemisch wird 1 1/2 Stunden bei 20 °C nachgerührt und anschließend mit 211 g (1 Mol) methanolischer Natriummethylat-Lösung und 80,6 g (1 Mol) Formamidin-Hydrochlorid versetzt. Man rührt die Mischung ohne Kühlung 18 Stunden nach, destilliert das Lösungsmittel im Vakuum ab und löst den Rückstand in 500 ml Wasser. Die Lösung wird im Vakuum bei 40 °C von restlichem organischen Lösungsmittel befreit und mit konzentrierter Salzsäure auf pH 2 eingestellt. Man rührt 2 Stunden bei 40 °C nach, gibt, verdünnte Natronlauge zu, bis pH 5 erreicht wird und kühlt dann auf 5 °C ab. Das ausgefallene Produkt wird abgesaugt und mit wenig kaltem Wasser gewaschen.

Man erhält so 63 g (56 % der Theorie) 4,5-Dihydroxy-pyrimidin in Form eines farblosen Pulvers mit dem Schmelzpunkt 253 °C.

Beispiel Ia/2

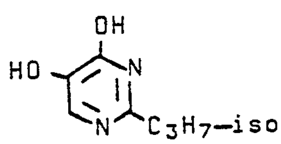

Aus einer Lösung von 168 g (1 Mol) 4-Hydroxy-5-methoxy-2-i-propyl-pyrimidin und 350 ml 48 %iger Bromwasserstoffsäure wird so lange Wasser abdestilliert, bis der Siedepunkt auf 120 °C angestiegen ist. Dann erhitzt man die Mischung 18 Stunden unter Rückfluß, destilliert im Vakuum die überschüssige

Bromwasserstoffsäure ab und löst den Rückstand in 300 ml Wasser. Die Lösung wird unter Kühlen bei 5-10 °C mit konzentrierter Natronlauge auf pH 5 eingestellt und das ausgefallene Produkt wird abgesaugt.

Man erhält auf diese Weise 149 g (97 % der Theorie) 4,5-Dihydroxy-2-i-propyl-pyrimidin in Form eines beigen Pulvers mit dem Schmelzpunkt 187 °C.

Analog Beispiel Ia/1 und Ia/2 werden z. B. die folgenden Verbindungen der Formel II erhalten :

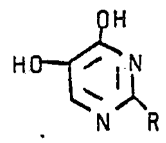

(II)

| Beispiel-Nr. | R | Fp (°C) |
|---|---|---|
| Ia/3 | tert.-$C_4H_9$ | 239 |
| Ia/4 | $CH_3$ | >250 |
| Ia/5 | $CH_2-SO_2-CH_3$ | 284 (Z) |

Ib Verfahren zur Herstellung der Ausgangsstoffe der Formel VI

Beispiel Ib/1

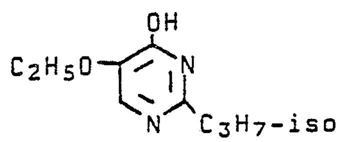

Eine Suspension aus 21,6 g (0,4 Mol) Natriummethylat, 24,5 g (0,2 Mol) iso-Butylamidinhydrochlorid und 32 g (0,2 Mol) Formylethoxyessigsäureethylester wird 6 Stunden unter Rückfluß erhitzt. Dann wird die Mischung eingedampft und der Rückstand in 200 ml Wasser aufgenommen. Die wäßrige Phase wird unter Eiskühlung mit konzentrierter Salzsäure bis zu einem pH 6 versetzt und anschließend zweimal mit je 200 ml Methylenchlorid extrahiert. Die vereinigten Methylenchloridextrakte werden über Natriumsulfat getrocknet und eingeengt.

Man erhält 22 g (60,5 % der Theorie) 5-Ethoxy-4-hydroxy-2-i-propyl-pyrimidin in Form farbloser Kristalle vom Schmelzpunkt 154 °C.

Analog Beispiel Ib/1 werden z. B. die folgenden Verbindungen der Formel VI erhalten :

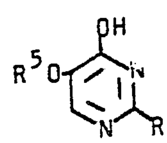

(VI)

Tabelle 6

| Beispiel-Nr. | $R^5$ | R | Fp (°C) |
|---|---|---|---|
| Ib/2 | i-$C_3H_7$ | i-$C_3H_7$ | 108 - 109 |
| Ib/3 | $C_2H_5$ | $CH_3$ | 160 |
| Ib/4 | $CH_3$ | i-$C_3H_7$ | 167 |
| Ib/5 | $CH_3$ | $CH_3$ | 206 |
| Ib/6 | $C_2H_5$ | $CH_3$ | 170 |
| Ib/7 | $CH_3$ | $C_2H_5$ | 168 |
| Ib/8 | $C_2H_5$ | H | 137 |

II Verfahren zur Herstellung der Verbindungen der allgemeinen Formel III (Verfahrensstufe (a))

Beispiel II/1

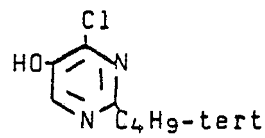

In eine Mischung aus 1 l Chloroform und 128 g (1,75 Mol) Dimethylformamid wird bei 5 °C 173 g (1,75 Mol) Phosgen eingeleitet. Unter weiterer Kühlung werden 168 g (1 Mol) 2-tert.-Butyl-4,5-dihydroxy-pyrimidin portionsweise zugegeben, so daß die Reaktionstemperatur von 20 °C nicht überschritten wird. Anschließend wird 3 Stunden bei 20 °C nachgerührt und danach werden unter Kühlung bei maximal 10 °C erst 200 ml Wasser und dann 1 000 g 45 %iger Natronlauge zugetropft. Die wäßrige Phase wird abgetrennt, im Vakuum von Chloroformresten befreit und dann bei 10 °C durch Zugabe von konzentrierter Salzsäure auf pH 6 eingestellt. Das ausgefallene Produkt wird abgesaugt und mit Wasser nachgewaschen.

Man erhält 172 g (92 % der Theorie) 2-tert.-Butyl-4-chlor-5-hydroxy-pyrimidin in Form eines schwach beigen Pulvers mit einem Schmelzpunkt von 108 °C.

Beispiel II/2

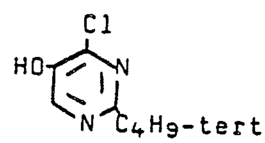

In eine Mischung aus 80 g Methylenchlorid und 14,6 g (0,2 Mol) Dimethylformamid wird bei 5 °C 19,8 g (0,2 Mol) Phosgen eingeleitet. Unter weiterer Kühlung werden 24 g (0,1 Mol) 2-tert.-Butyl-5-(1-ethoxy-ethoxy)-4-hydroxy-pyrimidin portionsweise zugegeben, so daß die Reaktionstemperatur 20 °C nicht übersteigt. Dann wird 3 Stunden bei 20 °C nachgerührt und unter Kühlung bei maximal 10 °C erst 45 ml Wasser und dann 45 g 45 %iger Natronlauge zugetropft. Die wäßrige Phase wird abgetrennt, im Vakuum von Methylenchloridresten befreit und dann bei 10 °C durch Zugabe von konzentrierter Salzäure auf pH 6 eingestellt. Das ausgefallene Produkt wird abgesaugt und mit Wasser nachgewaschen.

Man erhält 15,7 g (84 % der Theorie) 2-tert.-Butyl-4-chlor-5-hydroxy-pyrimidin in Form eines schwach beigen Pulvers mit dem Schmelzpunkt 108 °C.

Analog den Beispielen II/1 oder II/2 werden z. B. die folgenden Verbindungen der Formel III erhalten :

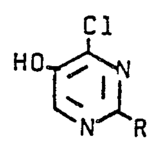

(III)

Tabelle 7

| Beispiel-Nr. | R | Physikal. Konstante |
|---|---|---|
| II/3 | $C_3H_7$-i | Fp: 105 - 107°C |
| II/4 | $CH_3$ | Fp: 73°C |

III Verfahren zur Herstellung der Verbindungen der allgemeinen Formel IV (Verfahrensstufe (b))

Beispiel III/1

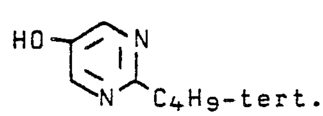

Eine Lösung von 186,5 g (1 Mol) 2-tert.-Butyl-4-chlor-5-hydroxy-pyrimidin und 84 g (2,1 Mol) Natriumhydroxid in 800 ml Wasser wird in Gegenwart von 15 g Raney-Nickel bei 50 °C und 10 bar Wasserstoffdruck hydriert. Nach Ende der Wasserstoffaufnahme wird der Katalysator abgesaugt. Das Filtrat wird mit konzentrierter Salzäure versetzt bis pH 4 erreicht wird. Das ausgefallene Produkt wird abgesaugt und mit Wasser nachgewaschen.

Man erhält auf diese Weise 110 g (77 % der Theorie) 2-tert.-Butyl-5-hydroxy-pyrimidin in Form eines farblosen Pulvers mit dem Schmelzpunkt 132 °C.

Analog dem Beispiel III/1 werden die folgenden Verbindungen der Formel IV erhalten

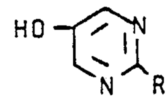

Tabelle 8

| Beispiel-Nr. | R | Fp [°C] |
|---|---|---|
| III/2 | $C_3H_7$-n | 117 |
| III/3 | H | 216 |
| III/4 | $CH_3$ | 173 |
| III/5 | $N(CH_3)_2$ | 164 |
| III/6 | $C_2H_5$ | 149 |
| III/7 | 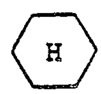 | 165 |
| III/8 | 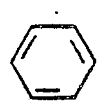 | 145 |

IV Verfahren zur Herstellung der Verbindungen der allgemeinen Formel I (Verfahrensstufe (c), welche aus der FR-A-2 365 577 bekannt ist)

Beispiel IV/1

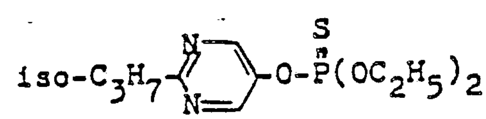

Ein Gemisch aus 300 ml Acetonitril, 13,8 g (0,1 Mol) 2-iso-Propyl-5-hydroxy-pyrimidin, 20,7 g (0,15 Mol) Kaliumcarbonat und 18,8 g (0,1 Mol) O,O-Diethylthionophosphorsäurediesterchlorid wird 2 Stunden bei 45 °C gerührt. Dann gießt man das Reaktionsgemisch in 400 ml Toluol und wäscht es zweimal mit je 300 ml Wasser. Die Toluollösung wird über Natriumsulfat getrocknet und im Vakuum eingedampft. Den Rückstand destilliert man im Hochvakuum an. Man erhält so 17,4 g (62 % der Theorie) O,O-Diethyl-O-[2-iso-propyl-pyrimidin(5)yl]-thionophosphorsäureester in Form eines braunen Öles mit dem Brechungsindex $n_D^{21}$ : 1,4970.

In analoger Weise können die foglenden Verbindungen der Formel

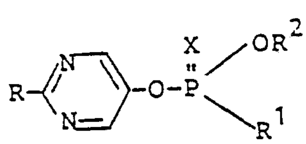

(I)

hergestellt werden :

Tabelle 9

| Beispiel Nr. | $R^2$ | $R^1$ | R | X | Ausbeute (% der Theorie) | Brechungsindex: |
|---|---|---|---|---|---|---|
| IV/2 | $C_3H_7$-iso | $CH_3$ | $C_3H_7$-iso | S | 74 | $n_D^{21}$: 1,5102 |
| IV/3 | $CH_3$ | $OCH_3$ | $C_3H_7$-iso | S | 66 | $n_D^{24}$: 1,5080 |
| IV/4 | $C_2H_5$ | $SC_3H_7$-n | $C_3H_7$-iso | S | 69 | $n_D^{26}$: 1,5284 |
| IV/5 | $C_2H_5$ | 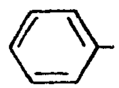 | $C_3H_7$-iso | S | 74 | $n_D^{26}$: 1,5570 |
| IV/6 | $C_2H_5$ | $OC_2H_5$ | $C_3H_7$-iso | O | 82 | $n_D^{32}$: 1,4630 |
| IV/7 | $C_2H_5$ | NH-$C_3H_7$-iso | $C_3H_7$-iso | S | 57 | $n_D^{32}$: 1,5057 |
| IV/8 | $C_3H_7$-n | $OC_2H_5$ | $C_3H_7$-iso | S | 73 | $n_D^{32}$: 1,4929 |
| IV/9 | $C_2H_5$ | $OC_2H_5$ | $CH_3$ | S | 92 | $n_D^{32}$: 1,4992 |
| IV/10 | $C_2H_5$ | $C_2H_5$ | $CH_3$ | S | 80 | $n_D^{32}$: 1,5169 |

Tabelle 9 (Fortsetzung)

| Beispiel Nr. | $R^2$ | $R^1$ | R | X | Ausbeute (% der Theorie) | Brechungsindex: |
|---|---|---|---|---|---|---|
| IV/11 | $C_2H_5$ | $OC_2H_5$ | 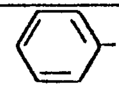 | S | 80 | $n_D^{32}$: 1,5643 |
| IV/12 | $C_2H_5$ | $C_2H_5$ | 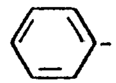 | S | 80 | $n_D^{32}$: 1,5827 |
| IV/13 | $C_2H_5$ | $OC_2H_5$ | H | S | 72 | $n_D^{32}$: 1,5028 |
| IV/14 | $C_2H_5$ | $OC_2H_5$ | $C_2H_5$ | S | 84 | $n_D^{20}$: 1,5014 |
| IV/15 | $C_2H_5$ | $OC_2H_5$ | $C_3H_7$-n | S | 60 | $n_D^{26}$: 1,4833 |
| IV/16 | $C_2H_5$ | $OC_2H_5$ | $C_4H_9$-n | S | 94 | $n_D^{21}$: 1,4958 |

Beispiel IV/17

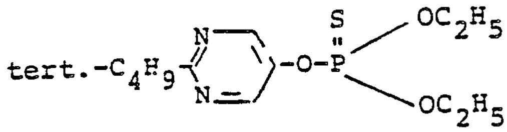

Eine Mischung aus 132 g (1 Mol) Glykolsäurebutylester, 72 g (1 Mol) Ethylvinylether und 0,3 g p-Toluolsulfonsäure läßt man unter schwachem Kühlen bei maximal 40 °C ausgereagieren. Nach Ende der exothermen Reaktion wird 2 Stunden bei 40 °C nachgerührt, dann gibt man bei 20 °C erst 90 g (1,5 Mol) Ameisensäuremethylester und anschießend unter leichtem Kühlen bei 20 °C 62 g (1,15 Mol) Natrium-methylat-Pulver portionsweise zu. Das Reaktionsgemisch wird 1 1/2 Stunden bei 20 °C nachgerührt und anschließend mit 211 g (1 Mol) methanolischer Natriummethylat-Lösung und 136,5 g (1 Mol) t.-Butyramidin-Hydrochlorid versetzt. Man rührt die Mischung ohne Kühlung 18 Stunden nach, gibt dann 120 ml konz. Salzsäure zu und rührt 1 Stunde bei ca. 50 °C nach. Dann wird das Lösungsmitel im Vakuum abdestilliert. Den festen Rückstand trocknet man an der Luft und gibt ihn bei 20 °C portionsweise zu einer Mischung aus 850 ml Chloroform, 109,5 g (1,5 Mol) Dimethylformamid und 148,5 g (1,5 Mol) Phosgen. Man rührt das Reaktionsgemisch 2 Stunden bei 20 °C nach und tropft dann unter Kühlen bei 0-10 °C eine Mischung aus 435 g 45 proz. Natronlauge und 800 ml Wasser zu. Die wäßrige Phase wird abgetrennt, im Vakuum von Chloroformresten befreit und anschließend in Gegenwart von 15 g Raney-Nickel bei 50 °C und 10 bar Wasserstoffdruck hydriert. Nach Ende der Wasserstoffaufnahme wird der Katalysator abgesaugt. Das Filtrat wird mit konzentrierter Salzsäure versetzt bis pH 4 erreicht ist. Das ausgefallene Produkt wird abgesaugt und mit Wasser gewaschen. Es wird nach dem Trocknen zu einer Mischung aus 300 ml Acetonitril, 124,2 g (0,9 Mol) Kaliumcarbonat und 116,9 g (0,62 Mol) O,O-Diethyl-thionophosphorsäurediesterchlorid gegeben und 2 Stunden bei 45 °C gerührt. Dann destilliert man das Lösungsmittel im Vakuum ab, löst den Rückstand in 400 ml Toluol und wäscht die Lösung zweimal mit je 300 ml Wasser. Die Toluollösung wird über Natriumsulfat getrocknet und im Vakuum eingedampft. Den Rückstand destilliert man im Hochvakuum an. Man erhält so 185 g (61 % der Theorie) O,O-Diethyl-O-[2-tert.-butyl-pyrimidin(5)yl]-thionophosphorsäureester in Form eines braunen Öles mit dem Brechungsindex $n_D^{26}$ : 1,4902.

Beispiel IV/18

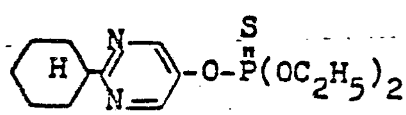

Ein Gemisch aus 300 ml Acetonitril, 17,8 g (0,1 Mol) 2-Cyclohexyl-5-hydroxy-pyrimidin, 20,7 g (0,15 Mol) Kaliumcarbonat und 18,8 g (0,1 Mol) O,O-Diethylthionophosphorsäurediesterchlorid wird 2 Stunden bei 45 °C gerührt. Dann gießt man das Reaktionsgemisch in 400 ml Toluol und wäscht es zweimal mit je 300 ml Wasser. Die Toluollösung wird über Natriumsulfat getrocknet und im Vakuum eingedampft. Den Rückstand destilliert man im Hochvakuum an. Man erhält so 21,7 g (66 % der Theorie) O,O-Diethyl-O-(2-cyclohexyl-pyrimidin(5)yl)-thionophosphorsäureester in Form eines braunen Öles mit dem Brechungsindex $n_D^{23}$ : 1,5158.

In analoger Weise können die folgenden Verbindungen der Formel

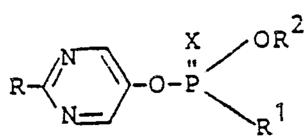

(I)

hergestellt werden :

(Siehe Tabelle 10 Seite 20 ff.)

Tabelle 10 (Fortsetzung)

| Beispiel Nr. | $R^2$ | $R^1$ | R | X | Ausbeute (% der Theorie) | Physikal. Daten (Brechungsindex; Schmelzpunkt °C) |
|---|---|---|---|---|---|---|
| IV/19 | $C_2H_5$ | $NH-C_3H_7$-iso | 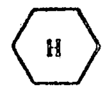 | S | 51 | $n_D^{23}$: 1,5246 |
| IV/20 | $CH_3$ | $OCH_3$ | 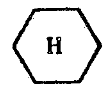 | S | 64 | $n_D^{23}$: 1,5287 |
| IV/21 | $C_2H_5$ | $OC_2H_5$ | 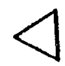 | S | 78 | $n_D^{24}$: 1,5142 |
| IV/22 | $C_2H_5$ | $NH-C_3H_7$-iso | 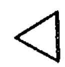 | S | 62 | 49 |
| IV/23 | $CH_3$ | $OCH_3$ | 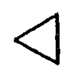 | S | 43 | $n_D^{24}$: 1,5390 |
| IV/24 | $C_3H_7$-n | $OC_2H_5$ | 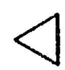 | S | 71 | $n_D^{25}$: 1,5128 |
| IV/25 | $C_2H_5$ | $NH-C_2H_5$ | 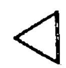 | S | 74 | $n_D^{26}$: 1,5310 |
| IV/26 | $C_2H_5$ | $OC_2H_5$ | 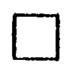 | S | | |
| IV/27 | $C_2H_5$ | $OC_2H_5$ | 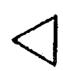 | S | | |

Tabelle 10 (Fortsetzung)

| Beispiel Nr. | $R^2$ | $R^1$ | R | X | Ausbeute (% der Theorie) | Physikal. Daten (Brechungsindex; Schmelzpunkt °C) |
|---|---|---|---|---|---|---|
| IV/28 | $C_2H_5$ | $OC_2H_5$ | 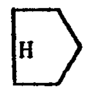 | S | 80 | $n_D^{23}$: 1,5164 |
| IV/29 | $C_2H_5$ | $OC_3H_7$-n | 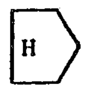 | S | | |
| IV/30 | $C_2H_5$ | $CH_3$ | 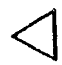 | S | 72 | $n_D^{25}$: 1,5428 |
| IV/31 | $C_2H_5$ | $OC_2H_5$ | 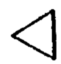 | O | | |
| IV/32 | $C_2H_5$ | NH-$C_3H_7$-iso | 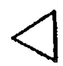 | O | | |
| IV/33 | $C_2H_5$ | 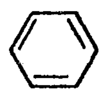 | 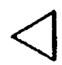 | S | 74 | $n_D^{25}$: 1,5815 |
| IV/34 | $C_2H_5$ | $SC_3H_7$-n | 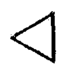 | S | | |
| IV/35 | $C_2H_5$ | 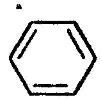 | H | S | | |
| IV/36 | $C_2H_5$ | NH-$C_2H_5$ | H | S | 66 | $n_D^{23}$: 1,5329 |

Tabelle 10 (Fortsetzung)

| Beispiel Nr. | $R^2$ | $R^1$ | R | X | Ausbeute (% der Theorie) | Physikal. Daten (Brechungsindex; Schmelzpunkt °C) |
|---|---|---|---|---|---|---|
| IV/37 | $C_2H_5$ | $SC_3H_7$ | 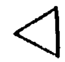 | O | | |
| IV/38 | $C_2H_5$ | $C_2H_5$ | 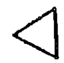 | S | | |
| IV/39 | $CH_3$ | $C_2H_5$ | 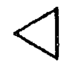 | S | | |
| IV/40 | $C_3H_7$-iso | $CH_3$ | 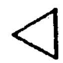 | S | 67 | $n_D^{26}$: 1,5233 |
| IV/41 | $CH_3$ | NH-$C_3H_7$-iso | 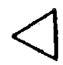 | S | | |
| IV/42 | $CH_3$ | NH-$CH_3$ | 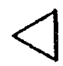 | S | 66 | $n_D^{26}$: 1,5460 |
| IV/43 | $C_2H_5$ | NH-$CH_3$ | 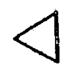 | S | | |
| IV/44 | $CH_3$ | NH-$C_2H_5$ | 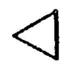 | S | | |
| IV/45 | $C_2H_5$ | NH-$C_3H_7$-iso | 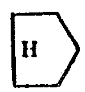 | S | 55 | $n_D^{23}$: 1,5247 |
| IV/46 | $C_2H_5$ | $OC_2H_5$ | 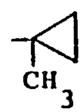 | S | | |

Beispiel IV/47

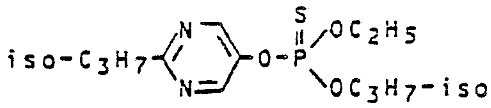

Ein Gemisch aus 300 ml Acetonitril, 13,8 g (0,1 Mol) 5-Hydroxy-2-iso-propyl-pyrimidin, 20,7 g (0,15 Mol) Kaliumcarbonat und 20,2 g (0,1 Mol) O-Ethyl-O-iso-propyl-thionophosphorsäurediester-chlorid wird 2 Stunden bei 45 °C gerührt. Dann gießt man das Reaktionsgemisch in 400 ml Toluol und wäscht es zweimal mit je 300 ml Wasser. Die Toluollösung wird über Natriumsulfat getrocknet und im Vakuum eingedampft. Den Rückstand destilliert man im Hochvakuum an.

Man erhält so 28 g (92 % der Theorie) O-Ethyl-O-iso-propyl-O-(2-iso-propyl-pyrimidin-5-yl)-thionophosphorsäureester in Form eines gelben Öles mit dem Brechungsindex $n_D^{23}$ : 1,4910.

In analoger Weise können die folgenden Verbindungen der Formel

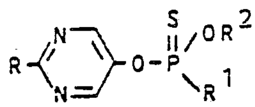

(I)

hergestellt werden :

(Siehe Tabelle 11 Seite 24 f.)

Tabelle 11

| Beispiel Nr. | R | $R^2$ | $R^1$ | Brechungsindex |
|---|---|---|---|---|
| IV/48 | $-C_3H_7$-iso | $-C_3H_7$-iso | $-OC_3H_7$-iso | $n_D^{20}$: 1,4869 |
| IV/49 | $-C_4H_9$-tert. | $-C_2H_5$ | $-OC_3H_7$-iso | $n_D^{20}$: 1,4917 |
| IV/50 | $-C_3H_7$-iso | $-C_2H_5$ | $-OC_4H_9$-sec. | $n_D^{20}$: 1,4960 |
| IV/51 | $-C_4H_9$-tert. | $-C_2H_5$ | $-OC_4H_9$-sec. | $n_D^{22}$: 1,4935 |
| IV/52 | $-C_4H_9$-tert. | $-C_3H_7$-iso | $-OC_3H_7$-iso | $n_D^{22}$: 1,4857 |
| IV/53 | 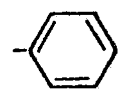 | $-C_2H_5$ | $-OC_3H_7$-iso | $n_D^{22}$: 1,5516 |
| IV/54 | $-C_4H_9$-tert. | $-C_2H_5$ | $-NHC_2H_5$ | $n_D^{21}$: 1,5100 |
| IV/55 | 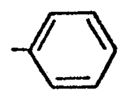 | $-C_2H_5$ | $-OC_4H_9$-sec. | |
| IV/56 | 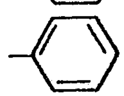 | $-C_3H_7$-iso | $-OC_3H_7$-iso | |
| IV/57 | $-C_3H_7$-iso | $-C_3H_7$-n | $-OC_3H_7$-n | $n_D^{23}$: 1,4915 |

**Patentansprüche**

1. Verfahren zur Herstellung der Verbindungen der allgemeinen Formel I

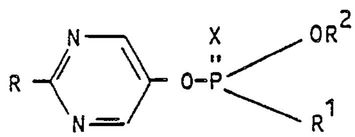

(I)

in welcher

R für Wasserstoff, für Alkoxy mit 1 bis 12 Kohlenstoffatomen, für Mono- oder Di-Alkylamino mit jeweils 1 bis 6 Kohlenstoffatomen im Alkylteil, für gegebenenfalls durch $C_1$-$C_4$-Alkoxy oder $C_1$-$C_4$-Alkylsulfonyl substituiertes Alkyl mit 1 bis 12 Kohlenstoffatomen, für gegebenenfalls durch $C_1$-$C_4$-Alkyl substituiertes Cycloalkyl mit 3 bis 8 Kohlenstoffatomen, und für gegebenenfalls durch $C_1$-$C_4$-Alkyl, $C_1$-$C_4$-Alkoxy oder $C_1$-$C_4$-Alkylsulfonyl substituiertes Aryl mit 6 bis 10 Kohlenstoffatomen steht,

$R^1$ für Reste aus der Reihe Alkyl, Alkoxy, Alkylthio mit jeweils 1 bis 6 Kohlenstoffatomen, für Mono- oder Dialkylamino mit 1 bis 6 Kohlenstoffatomen je Alkylgruppe und für Phenyl steht,

$R^2$ für Alkyl mit 1 bis 6 Kohlenstoffatomen steht,

wobei die Reste $R^1$ und $R^2$ durch 1 bis 3 gleiche oder verschiedene Substituenten aus der Reihe Alkyl (gilt nicht für die Fälle, in welchen $R^1$ bzw. $R^2$ für Alkyl steht), Alkoxy oder Alkylthio mit jeweils 1 bis 4 Kohlenstoffatomen oder Halogen, Cyano und Nitro substituiert sein können, und

X für Sauerstoff oder Schwefel steht,

durch die Umsetzung der Verbindungen der allgemeinen Formel IV

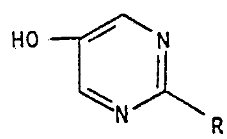

(IV)

in welcher R die oben angegebene Bedeutung hat, mit Verbindungen der allgemeinen Formel V

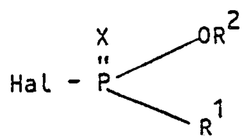

(V)

in welcher

Hal für Halogen steht und

X, $R^1$ und $R^2$ die oben angegebene Bedeutung haben,

gegebenenfalls in Gegenwart eines Säurebindemittels und gegebenenfalls in Gegenwart eines Lösungsmittels, mit nachfolgender Isolierung der Verbindungen der allgemeinen Formel I (Schritt (c)), dadurch gekennzeichnet, daß man die Verbindungen der allgemeinen Formel IV erhält, indem man

(a) Verbindungen der allgemeinen Formel II

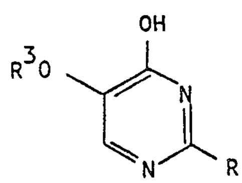

(II)

in welcher

R die oben angegebene Bedeutung hat,

$R^3$ für Wasserstoff oder eine Gruppierung

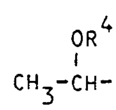

steht, wobei

$R^4$ für Alkyl mit 1 bis 4 Kohlenstoffatomen steht,

mit Halogenierungsmitteln in Gegenwart von N,N-disubstituierten Amiden und gegebenenfalls in

Gegenwart von Verdünnungsmitteln bei Temperaturen zwischen 10 °C und 80 °C zu den Verbindungen der allgemeinen Formel III

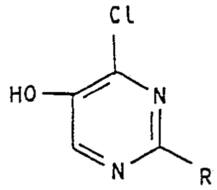

(III)

in welcher R die oben angegebene Bedeutung hat, umsetzt und anschließend

(b) die Verbindungen der allgemeinen Formel III, gegebenenfalls nach ihrer Isolierung, mit Wasserstoff in Gegenwart von Hydrierungskatalysatoren, in Gegenwart von Säureakzeptoren und in Gegenwart von Verdünnungsmitteln, bei Temperaturen zwischen 20 °C und 150 °C zu den Verbindungen der allgemeinen Formel IV umsetzt und diese zur weiteren Umsetzung mit den Verbindungen der allgemeinen Formel V gegebenenfalls isoliert.

2. Verfahren gemäß Anspruch 1, dadurch gekennzeichnet, daß R für $C_1$-$C_4$-Alkyl, $C_3$-$C_6$-Cycloalkyl oder Phenyl, $R^1$ für $C_1$-$C_4$-Alkoxy, $R^2$ für $C_1$-$C_4$-Alkyl und X für Schwefel stehen.

3. Verbindungen der allgemeinen Formel VII

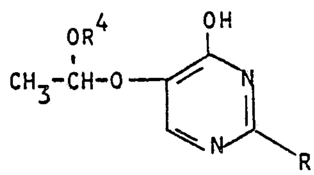

(VII)

in welcher

R für Wasserstoff, für Alkoxy mit 1 bis 12 Kohlenstoffatomen, für Mono- oder Di-Alkylamino mit jeweils 1 bis 6 Kohlenstoffatomen im Alkylteil, für gegebenenfalls durch $C_1$-$C_4$-Alkoxy oder $C_1$-$C_4$-Alkylsulfonyl substituiertes Alkyl mit 1 bis 12 Kohlenstoffatomen, für gegebenenfalls durch $C_1$-$C_4$-Alkyl substituiertes Cycloalkyl mit 3 bis 8 Kohlenstoffatomen, und für gegebenenfalls durch $C_1$-$C_4$-Alkyl, $C_1$-$C_4$-Alkoxy oder $C_1$-$C_4$-Alkylsulfonyl substituiertes Aryl mit 6 bis 10 Kohlenstoffatomen steht und

$R^4$ für Alkyl mit 1 bis 4 Kohlenstoffatomen steht.

4. Verfahren zur Herstellung der Verbindungen der allgemeinen Formel VII gemäß Anspruch 3, dadurch gekennzeichnet, daß man Hydroxyessigsäureester der allgemeinen Formel VIII

$$HO{-}CH_2COOR^6 \qquad (VIII)$$

in welcher $R^6$ für $C_1$-$C_4$-Alkyl steht, mit Vinylethern der Formel IX

$$CH_2 = CH\text{-}OR^4 \qquad (IX)$$

in welcher $R^4$ die in Anspruch 3 angegebene Bedeutung hat, in Gegenwart von Katalysatoren umsetzt, weiterhin mit Ameisensäureestern der Formel X

$$HCOOR^7 \qquad (X)$$

in welcher $R^7$ für $C_1$-$C_4$-Alkyl steht, in Gegenwart einer Base, reagieren läßt und anschließend mit Amidin-Hydrochloriden der allgemeinen Formel XI

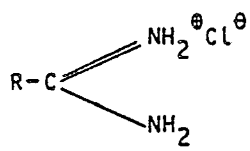

(XI)

in welcher R die in Anspruch 3 angegebene Bedeutung hat, in Gegenwart von Basen und in Gegenwart von Verdünnungsmitteln bei Temperaturen zwischen 15 °C und 60 °C umsetzt.

5. Verbindungen der allgemeinen Formel III

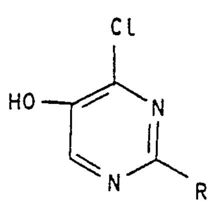

(III)

in welcher R für Wasserstoff, für Alkoxy mit 1 bis 12 Kohlenstoffatomen, für Mono- oder Di-Alkylamino mit jeweils 1 bis 6 Kohlenstoffatomen im Alkylteil, für gegebenenfalls durch $C_1$-$C_4$-Alkoxy oder $C_1$-$C_4$-Alkylsulfonyl substituiertes Alkyl mit 1 bis 12 Kohlenstoffatomen, für gegebenenfalls durch $C_1$-$C_4$-Alkyl substituiertes Cycloalkyl mit 3 bis 8 Kohlenstoffatomen, und für gegebenfalls durch $C_1$-$C_4$-Alkyl, $C_1$-$C_4$-Alkoxy oder $C_1$-$C_4$-Alkylsulfonyl substituiertes Aryl mit 6 bis 10 Kohlenstoffatomen steht.

6. Verfahren zur Herstellung der Verbindungen der allgemeinen Formel III gemäß Anspruch 5, dadurch gekennzeichnet, daß man Verbindungen der allgemeinen Formel II

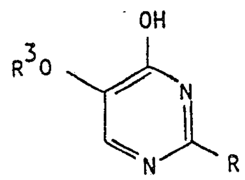

(II)

in welcher

R die in Anspruch 5 angegebene Bedeutung hat, und

$R^3$ für Wasserstoff oder die Gruppe

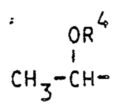

steht, in welcher

$R^4$ Alkyl mit 1 bis 4 Kohlenstoffatomen bedeutet,

mit Halogenierungsmitteln in Gegenwart von N,N-disubstituierten Amiden und gegebenenfalls in Gegenwart von Verdünnungsmitteln bei Temperaturen zwischen 10 °C und 80 °C umsetzt.

7. Verfahren zur Herstellung von Verbindungen der allgemeinen Formel IV

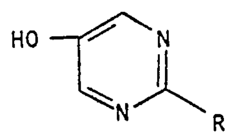

(IV)

in welcher R für Wasserstoff, für Alkoxy mit 1 bis 12 Kohlenstoffatomen, für Mono- oder Di-Alkylamino mit jeweils 1 bis 6 Kohlenstoffatomen im Alkylteil, für gegebenenfalls durch $C_1$-$C_4$-Alkoxy oder $C_1$-$C_4$-Alkylsulfonyl substituiertes Alkyl mit 1 bis 12 Kohlenstoffatomen, für gegebenenfalls durch $C_1$-$C_4$-Alkyl substituiertes Cycloalkyl mit 3 bis 8 Kohlenstoffatomen, und für gegenbenenfalls durch $C_1$-$C_4$-Alkyl, $C_1$-$C_4$-alkoxy oder $C_1$-$C_4$-Alkylsulfonyl substituiertes Aryl mit 6 bis 10 Kohlenstoffatomen steht, dadurch gekennzeichnet, daß man Verbindungen der allgemeinen Formel III

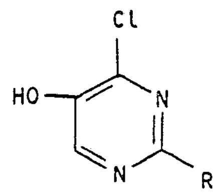

(III)

in welcher R die oben angegebene Bedeutung hat, mit Wasserstoff in Gegenwart von Hydrierungskatalysatoren, in Gegenwart von Säureakzeptoren und in Gegenwart von Verdünnungsmitteln, bei Temperaturen zwischen 20 °C und 150 °C umsetzt.

8. Verbindungen der allgemeinen Formeln VII und III gemäß den Ansprüchen 3 und 5, in welchen R für $C_1$-$C_4$-Alkyl steht.

9. Verfahren gemäß den Ansprüchen 4, 6 und 7, wobei R in den Verbindungen der allgemeinen Formeln XI, II, IV und III für $C_1$-$C_4$-Alkyl steht.

**Claims**

1. Process for the preparation of compounds of the general formula I

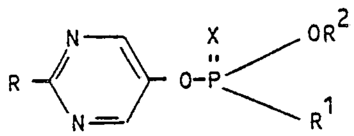

(I)

in which

R represents hydrogen, alkoxy having 1 to 12 carbon atoms, monoalkylamino or dialkylamino having 1 to 6 carbon atoms in each alkyl moiety, alkyl which has 1 to 12 carbon atoms and is optionally substituted by $C_1$-$C_2$-alkoxy or $C_1$-$C_4$-alkylsulphonyl, cycloalkyl which has 3 to 8 carbon atoms and is optionally substituted by $C_1$-$C_4$-alkyl, and aryl which has 6 to 10 carbon atoms and is optionally substituted by $C_1$-$C_4$-alkyl, $C_1$-$C_4$-alkoxy or$C_1$-$C_4$-alkylsulphonyl,

$R^1$ represents radicals from the series comprising alkyl, alkoxy, alkylthio, in each case having 1 to 6 carbon atoms, monoalkylamino or dialkylamino which has 1 to 6 carbon atoms in each alkyl group, and phenyl,

$R^2$ represents alkyl having 1 to 6 carbon atoms, where the radicals $R^1$ and $R^2$ can be substituted by 1 to 3 identical or different substituents from the series comprising alkyl (does not apply to the cases where $R^1$ or $R^2$ represents alkyl), alkoxy and alkylthio having in each case 1 to 4 carbon atoms, and halogen, cyano and nitro, and

X represents oxygen or sulphur, by the reaction of the compounds of the general formula IV

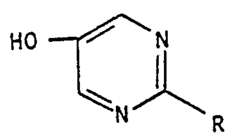

(IV)

in which R has the abovementioned meaning, with compounds of the general formula V

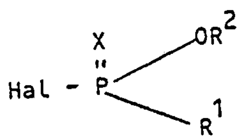

(V)

in which

Hal represents halogen and

X, $R^1$ and $R^2$ have the abovementioned meaning,

where appropriate in the presence of an acid-binding agent and where appropriate in the presence of a solvent, with subsequent isolation of the compounds of the general formula I (step (c)), characterized in that the compounds of the general formula IV are obtained when

(a) compounds of the general formula II

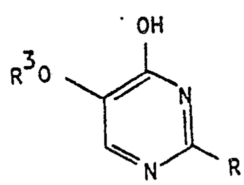

(II)

in which

R has the abovementioned meaning,

$R_3$ represents hydrogen or a group

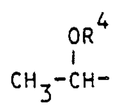

$R^4$ representing alkyl having 1 to 4 carbon atoms, are reacted with halogenating agents in the presence of N,N-disubstituted amides and, where appropriate, in the presence of diluents, at temperatures between 10 °C and 80 °C, to give the compounds of the general formula III

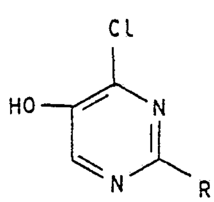

(III)

in which R has the abovementioned meaning, and then

(b) the compounds of the general formula III, where appropriate after their isolation, are reacted with hydrogen in the presence of hydrogenation catalysts, in the presence of acid acceptors and in the presence of diluents, at temperatures between 20 °C and 150 °C, to give the compounds of the general formula IV and these are, where appropriate, isolated for further reaction with the compounds of the general formula V.

2. Process according to Claim 1, characterized in that R represents $C_1$-$C_4$-alkyl, $C_3$-$C_6$-cycloalkyl or phenyl, $R^1$ represents $C_1$-$C_4$-alkoxy, $R^2$ represents $C_1$-$C_4$-alkyl and X represents sulphur.

3. Compounds of the general formula VII

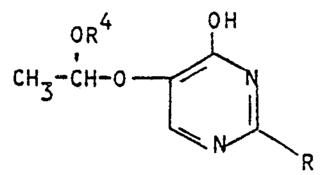

(VII)

in which

R represents hydrogen, alkoxy having 1 to 12 carbon atoms, monoalkylamino or dialkylamino having 1 to 6 carbon atoms in each alkyl moiety, alkyl which has 1 to 12 carbon atoms and is optionally substituted by $C_1$-$C_4$-alkoxy or $C_1$-$C_4$-alkylsulphonyl, cycloalkyl which has 3 to 8 carbon atoms and is optionally substituted by $C_1$-$C_4$-alkyl, and aryl which has 6 to 10 carbon atoms and is optionally substituted by $C_1$-$C_4$-alkyl, $C_1$-$C_4$-alkoxy or $C_1$-$C_4$-alkylsulphonyl and

$R^4$ represents alkyl having 1 to 4 carbon atoms.

4. Process for the preparation of the compounds of the general formula VII according to Claim 3, characterized in that hydroxyacetic esters of the general formula VIII

$$HO—CH_2COOR^6 \qquad (VIII)$$

in which $R^6$ represents $C_1$-$C_4$-alkyl, are reacted with vinyl ethers of the formula IX

$$CH_2 = CH—OR^4 \qquad (IX)$$

in which $R^4$ has the meaning indicated in Claim 3, in the presence of catalysts, and are reacted further with formic esters of the formula X

$$HCOOR^7 \qquad (X)$$

in which $R^7$ represents $C_1$-$C_4$-alkyl, in the presence of a base, and are then reacted with amidine hydrochlorides of the general formula XI

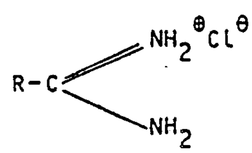

(XI)

in which R has the meaning indicated in Claim 3, in the presence of bases and in the presence of diluents, at temperatures between 15 °C and 60 °C.

5. Compounds of the general formula III

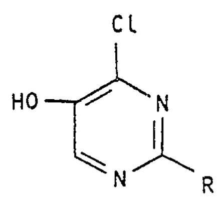

(III)

in which R represents hydrogen, alkoxy having 1 to 12 carbon atoms, monoalkylamino or dialkylamino having 1 to 6 carbon atoms in each alkyl moiety, alkyl which has 1 to 12 carbon atoms and is optionally substituted by $C_1$-$C_4$-alkoxy or $C_1$-$C_4$-alkylsulphonyl, cycloalkyl which has 3 to 8 carbon atoms and is optionally substituted by $C_1$-$C_4$-alkyl, and aryl which has 6 to 10 carbon atoms and is optionally substituted by $C_1$-$C_4$-alkyl, $C_1$-$C_4$-alkoxy or $C_1$-$C_4$-alkylsulphonyl.

6. Process for the preparation of the compounds of the general formula III according to Claim 5, characterized in that compounds of the general formula II

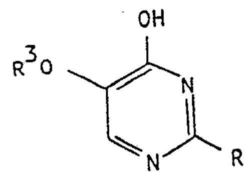

(II)

in which

R has the meaning indicated in Claim 5, and

$R_3$ represents hydrogen or the group

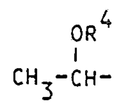

in which

$R^4$ denotes alkyl having 1 to 4 carbon atoms, are reacted with halogenating agents in the presence of N,N-disubstituted amides and, where appropriate, in the presence of diluents, at temperatures between 10 °C and 80 °C.

7. Process for the preparation of compounds of the general formula IV

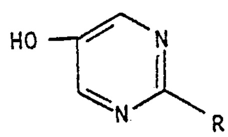

(IV)

in which R represents hydrogen, alkoxy having 1 to 12 carbon atoms, monoalkylamino or dialkylamino having 1 to 6 carbon atoms in each alkyl moiety, alkyl which has 1 to 12 carbon atoms and is optionally substituted by $C_1$-$C_4$-alkoxy or $C_1$-$C_4$-alkylsulphonyl, cycloalkyl which has 3 to 8 carbon atoms and is optionally substituted by $C_1$-$C_4$-alkyl, and aryl which hasd 6 to 10 carbon atoms and is optionaly substituted by $C_1$-$C_4$-alkyl, $C_1$-$C_4$-alkoxy or $C_1$-$C_4$-alkylsulphonyl,
characterized in that compounds of the general formula III

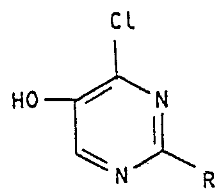

(III)

in which R has the abovementioned meaning, are reacted with hydrogen in the presence of hydrogenation catalysts, in the presence of acid acceptors and in the presence of diluents, at tempeatures between 20 °C and 150 °C.

8. Compounds of the general formulae VII and III according to Claims 3 and 5, in which R represents $C_1$-$C_4$-alkyl.

9. Process according to Claims 4, 6 and 7, R in the compounds of the general formulae XI, II, IV and III representing $C_1$-$C_4$-alkyl.

**Revendications**

1. Procédé de préparation des composés de formule générale I

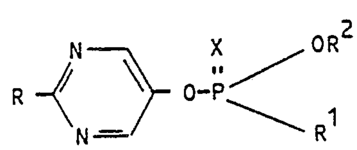

(I)

dans laquelle

R représente l'hydrogène, un groupe alcoxy en $C_1$-$C_{12}$, un groupe mono- ou dialkylamino contenant chacun 1 à 6 atomes de carbone par partie alkyle, un groupe alkyle en $C_1$-$C_{12}$ éventuellement substitué

par un groupe alcoxy en $C_1$-$C_4$ ou alkylsulfonyle en $C_1$-$C_4$, un groupe cycloalkyle en $C_3$-$C_8$ éventuellement substitué par un groupe alkyle en $C_1$-$C_4$, et un groupe aryle en $C_6$-$C_{10}$ éventuellement substitué par un groupe alkyle en $C_1$-$C_4$, alcoxy en $C_1$-$C_4$ ou alkyl-sulfonyle en $C_1$-$C_4$,

$R^1$ représente un groupe choisi parmi les groupes alkyle, alcoxy, alkylthio contenant chacun 1 à 6 atomes de carbone, les groupes mono- ou dialkylamino contenant 1 à 6 atomes de carbone par groupe alkyle, et les groupes phényle,

$R^2$ représente un groupe alkyle en $C_1$-$C_6$, les groupes $R^1$ et $R^2$ pouvant porter 1 à 3 substituants identiques ou différents choisis parmi les groupes alkyle (sauf dans les cas où $R^1$ ou $R^2$ représente un groupe alkyle), alcoxy ou alkylthio contenant chacun 1 à 4 atomes de carbone ou les halogènes, les groupes cyano et nitro, et

X représente l'oxygène ou le soufre, par réaction des composés de formule générale IV

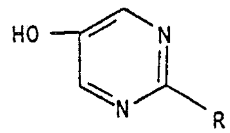

(IV)

dans laquelle R a les significations indiquées ci-dessus, avec des composés de formule générale V

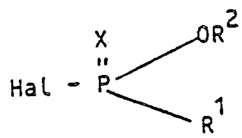

(V)

dans laquelle

Hal représente un halogène, et

X, $R^1$ et $R^2$ ont les significations indiquées ci-dessus,

éventuellement en présence d'un accepteur d'acide et le cas échéant en présence d'un solvant, avec isolement subséquent des composés de formule générale I (stade opératoire c)),

caractérisé en ce que l'on obtient les composés de formule générale IV

a) en faisant réagir des composés de formule générale II

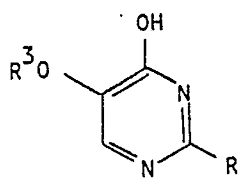

(II)

dans laquelle

R a les significations indiquées ci-dessus,

$R^3$ représente l'hydrogène ou un groupement

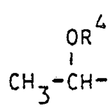

dans lequel $R^4$ représente un groupe alkyle en $C_1$-$C_4$, avec des agents halogénants en présence d'amides N,N-disubstitués et le cas échéant en présence de diluants à des températures de 10 à 80 °C, ce qui donne des composés de formule générale III

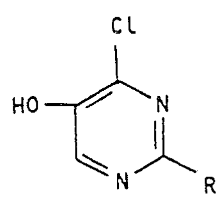

(III)

dans laquelle R a les significations indiquées ci-dessus, puis

b) en faisant réagir les composés de formule générale III, le cas échéant après les avoir isolés, avec l'hydrogène en présence de catalyseurs d'hydrogénation, en présence d'accepteurs d'acides et en présence de diluants, à des températures de 20 à 150 °C, ce qui donne les composés de formule générale IV qu'on isole le cas échéant avant de les faire ensuite réagir avec les composés de formule générale V.

2. Procédé selon la revendication 1, caractérisé en ce que R représente un groupe alkyle en $C_1$-$C_4$, cycloalkyle en $C_3$-$C_6$ ou phényle, $R^1$ représente un groupe alcoxy en $C_1$-$C_4$, $R^2$ représente un groupe alkyle en $C_1$-$C_4$ et X représente le soufre.

3. Composés de formule générale VII

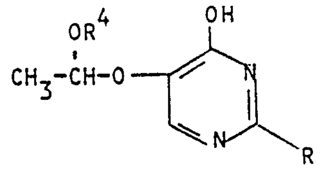

(VII)

dans laquelle

R représente l'hydrogène, un groupe alcoxy en $C_1$-$C_{12}$, un groupe mono- ou di-alkylamino contenant chacun 1 à 6 atomes de carbone par partie alkyle, un groupe alkyle en $C_1$-$C_{12}$ éventuellement substitué par un groupe alcoxy en $C_1$-$C_4$ ou alkylsulfonyle en $C_1$-$C_4$, un groupe cycloalkyle en $C_3$-$C_8$ éventuellement substitué par un groupe alkyle en $C_1$-$C_4$, et un groupe aryle en $C_6$-$C_{10}$ éventuellement substitué par un groupe alkyle en $C_1$-$C_4$, alcoxy en $C_1$-$C_4$ ou alkylsulfonyle en $C_1$-$C_4$, et

$R^4$ représente un groupe alkyle en $C_1$-$C_4$.

4. Procédé de préparation des composés de formule générale VII selon la revendication 3, caractérisé en ce que l'on fait réagir des esters hydroxyacétiques de formule générale VIII

$$HO\text{—}CH_2COOR^6 \qquad (VIII)$$

dans laquelle $R^6$ représente un groupe alkyle en $C_1$-$C_4$, avec des éthers vinyliques de formule IX

$$CH_2 = CH\text{—}OR^4 \qquad (IX)$$

dans laquelle $R^4$ a les significations indiquées dans la revendication 3, en présence de catalyseurs, puis avec des esters formiques de formule X

$$HCOOR^7 \qquad (X)$$

dans laquelle $R^7$ représente un groupe alkyle en $C_1$-$C_4$, en présence d'une base, puis avec des chlorhydrates d'amidines de formule générale XI

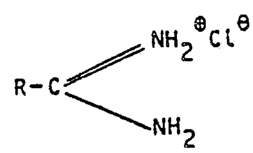

(XI)

dans laquelle R a les significations indiquées dans la revendication 3, en présence de bases et en présence de diluants, à des températures de 15 à 60 °C.

5. Composés de formule générale III

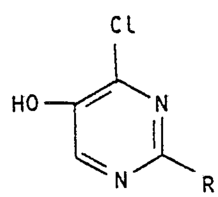

(III)

dans laquelle R représente l'hydrogène, un groupe alcoxy en $C_1$-$C_{12}$, un groupe mono- ou di-alkylamino contenant chacun 1 à 6 atomes de carbone par partie alkyle, un groupe alkyle en $C_1$-$C_{12}$ éventuellement substitué par un groupe alcoxy en $C_1$-$C_4$ ou alkylsulfonyle en $C_1$-$C_4$, un groupe cycloalkyle en $C_3$-$C_8$ éventuellement substitué par un groupe alkyle en $C_1$-$C_4$, et un groupe aryle en $C_6$-$C_{10}$ éventuellement substitué par un groupe alkyle en $C_1$-$C_4$, alcoxy en $C_1$-$C_4$ ou alkylsulfonyle en $C_1$-$C_4$.

6. Procédé de préparation des composés de formule générale III selon la revendication 5, caractérisé en ce que l'on fait réagir des composés de formule générale II

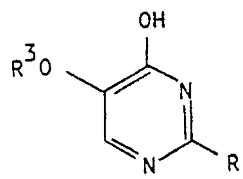

(II)

dans laquelle

R a les significations indiquées dans la revendication 5, et

$R^3$ représente l'hydrogène ou le groupe

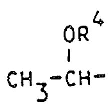

dans lequel $R^4$ représente un groupe alkyle en $C_1$-$C_4$, avec des agents halogénants en présence d'amides N,N-disubstitués et le cas échéant en présence de diluants, à des températures de 10 à 80 °C.

7. Procédé de préparation des composés de formule générale IV

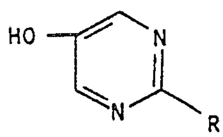

(IV)

dans laquelle R représente l'hydrogène, un groupe alcoxy en $C_1$-$C_{12}$, un groupe mono- ou dialkylamino contenant chacun 1 à 6 atomes de carbone par partie alkyle, un groupe alkyle cn $C_1$-$C_{12}$ éventuellement substitué par un groupe alcoxy en $C_1$-$C_4$ ou alkylsulfonyle en $C_1$-$C_4$, un groupe cycloalkyle en $C_3$-$C_8$ éventuellement substitué par un groupe alkyle en $C_1$-$C_4$, et un groupe aryle en $C_6$-$C_{10}$ éventuellement substitué par un groupe alkyle en $C_1$-$C_4$, alcoxy en $C_1$-$C_4$ ou alkylsufonyle en $C_1$-$C_4$,

caractérisé en ce que l'on fait réagir des composés de formule générale III

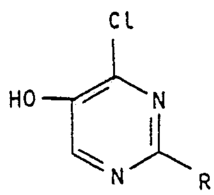

(III)

dans laquelle R a les significations indiquées ci-dessus, avec l'hydrogène en présence de catalyseurs d'hydrogénation, en présence d'accepteurs d'acides et en présence de diluants, à des températures de 20 à 150 °C.

8. Composés de formules générales VII et III selon les revendications 3 et 5, dans lesquels R représente un groupe alkyle en $C_1$-$C_4$.

9. Procédé selon les revendications 4, 6 et 7, dans lequel R des composés de formules générales XI, II, IV et III représente un groupe alkyle en $C_1$-$C_4$.